# EUROPEAN PATENT SPECIFICATION

(11) **EP 0 314 127 B1**
(45) Date of publication and mention of the grant of the patent: **03.08.1994**
(21) Application number: 88117918.8
(22) Date of filing: 27.10.1988
(51) Int. Cl.: C07D 207/46, C07D 207/40, C07K 5/10, C07K 5/08, G01N 33/535, G01N 33/547, C07K 15/00

(54) **Heterobifunctional coupling agents**
Heterobifunktionelle Kupplungsmittel
Agents de couplage hétérobifonctionnels

(30) Priority: 30.10.1987 US 114930; 22.09.1988 US 246971; 11.10.1988 US 254288
(43) Date of publication of application: 03.05.1989
(73) Proprietor: ABBOTT LABORATORIES, Abbott Park, Illinois 60064-3500 (US)
(72) Inventor: Bieniarz, Christopher, Highland Park Illinois 60035 (US); Barnes, Grady, Lindenhurst Illinois 60046 (US); Welch, Christopher Joseph, Chicago Illinois 60626 (US); Schlesinger, Carol A., Wheeling Illinois 60090 (US)
(74) Representative: Modiano, Guido, Dr.-Ing.

(56) References cited:
- EP-A- 0 109 653
- EP-A- 0 158 291
- EP-A- 0 178 125
- JP-A-59 176 675
- JP-A-60 146 154
- CHEM. PHARM. BULL., vol. 29, no. 4, 1981, pages 1130-1135; T. KITAGAWA et al.:"Preparation and characterization of hetero-bifunctional cross-linking reagentsfor protein modifications"
- CHEMICAL ABSTRACTS, vol. 90, no. 21, 21st May 1979, page 3, abstract no.161838c, Columbus, Ohio, US; H. KATO et al.: "Enzyme immunoassay ofpenicillin",
- J. BIOCHEM., vol. 92, no. 5, 1982, pages 1413-1424; S. YOSHITAKE et al.: "Mildand efficient conjugation of rabbit Fab' and horseradish peroxidase using amaleimide compound and its use for enzyme immunoassay"

## Description

### Background of the Invention

The present invention relates to coupling agents for covalent conjugation of proteins to proteins or proteins to solid phases for use in diagnostic assays.

It is well established that a protein such as an enzyme can be conjugated via a linker group to another protein such as an antibody to form a conjugate which can be used in enzyme immunoassays (EIAs). However, protein-protein conjugates have been accompanied often by reduced or altered activity of at least one of the proteins.

In the past, protein-protein conjugates have been formed using heterobifunctional reagents which are quite short in comparison with the protein molecules being conjugated. It is believed that by restricting the conformational freedom of a protein, the function of the protein (e.g., initial rate, turnover number, and Km for an enzyme, or affinity for an antibody) can be adversely affected, thereby rendering the individual components of an enzyme-antibody conjugate less active than their unconjugated counterparts. This has been a problem which has limited the bioactivity of enzyme-antibody conjugates and their application to immunoassays.

Linkage of proteins to solid phases has also been accompanied by frequent problems in the past. For instance, in diagnostic assays, the reaction between a specific binding member and its complement is often employed to detect whether (and in some assays, how much) specific binding member or complement is present in a sample. In one type of diagnostic assay, a specific binding member (e.g. an antibody) is detected in a sample by introducing its complement (e.g. an antigen) into the sample and determining if any reaction occurs between the two reagents. Alternatively, the complement itself can be detected in a sample by introducing the specific binding member into the sample and determining whether any reaction occurs. Because it is often difficult to detect whether any reaction has occurred, a second specific binding member may be added to the sample. The second specific binding member can react either with the first specific binding member or its complement, and the second member bears a detectable label. Of course, it is impossible to determine beforehand how much labelled second specific binding member must be added because it is unknown how much, if any, of the substance to be detected is in the sample.

Thus, the labelled specific binding member is added in excess of the maximum concentration of the substance typically found in such samples. However, the labelled specific binding member which does not bind with the substance must be separated from the sample so that only the bound labelled member is detected, indicating that the substance is indeed in the sample.

A common approach to separate bound from unbound labelled member is to employ solid phase separation. A typical example of such separation involves linking the first specific binding member (in the case of assays for complement to the first member) or complement (in the case of assays for specific binding member) to a solid phase (such as microparticles) which can be separated from the sample, for example, by filtration or gravity sedimentation. The label associated either with the solid phase or still in the sample is proportional to the amount of substance to be detected in the original sample.

Other variations to this general solid phase separation scheme have been developed, but most such schemes involve the binding of the substance to be analyzed to a specific binding member linked to a solid phase. This binding is crucial to assay performance. However, the linkage between the solid phase and the specific binding member can affect binding. An example will illustrate the point. Antibodies have extremely specific structural, spacial, and polar configurations which endow them with the ability to recognize and bind to one type of analyte, and virtually none other. When antibodies are employed in assays for the detection of antigens, antibodies can be linked to solid phases. However, the proximity of the solid phase to the antibody can block sites where antigen binds. Alternatively, the linkage (usually covalent) between the antibody and solid phase can alter the structure (conformation) of the antibody so that the linkage may deleteriously affect binding of the antibody to the analyte. The same situation holds for conjugation of analytes, particularly proteins, to solid phases. The analyte conformation can change upon conjugation so the free antibody in the sample can no longer recognize it.

Covalent attachment of proteins to solid phases using heterobifunctional reagents has been accomplished with mixed results in the past. In some cases the proteins were directly conjugated to the solid phase. Generally, the connecting tether has been quite short in comparison with the size of the bound protein. This is disadvantageous in that the bound protein can still be hindered in performing its biological function due to steric crowding, inaccessability of binding sites, etc.

This has been a problem which has limited the bioactivity and stability of derivatized solid phases in the past.

As relevant background art to the present invention, the following documents may be cited: Chem. Pharm. Bull., 1981, vol. 29, no. 4, pages 1130-1135 discloses N-(maleimido benzoyloxy)-succinamide cross-linking reagents. JP-A-60 146 154 discloses the use of succinimidyl-4-(N-maleimidomethyl)cyclohexane-1-carboxylate (SMCC) cross-linked to osteocarcin. JP-A-59 176 675 discloses N-(maleimide arnoyloxy)succinamide as a cross-linker with an antigen or antibody and a marking enzyme. Chemical Abstracts, 1979, vol. 90, no. 21, abstr. 161838c discloses N-(3-maleimido propionylglycyloxy)succinimide as a cross-linker of ampicillin. EP-A-0 158 291 discloses N-(maleimidomethylcyclohexylene)succinimide as a cross-linker between an anti-CEA antibody and peroxidase. EP-A-0 178 125 discloses heterobifunctional coupling agents useful in joining radionuclide metal ions to antibody fragments. EP-A-0 109 653 discloses linking fibrin-adsorbable protein with urokinase by the use of heterobifunctional reagents. J. Biochem., 1982, vol. 92, no. 5, pages 1413-1424 discloses the N-hydroxysuccinimide ester of N-(4-carboxycyclohexylmethyl) maleimide as a heterobifunctional cross-linker.

### Summary of the Invention

The present invention includes heterobifunctional coupling agents of formula I: 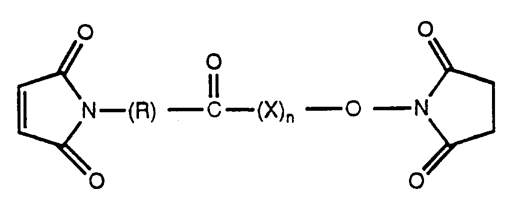
wherein X is an amino acid having from three to ten carbon atoms in a straight chain.

R is an alkyl, cycloalkyl, an alkyl-cycloalkyl, or phenyl; and n is from one to ten.

This invention also involves conjugates of solid phases with the novel linking groups above which can be used to link solid phases to substances such as proteins, antibodies, antigens and the like. These conjugates are hydrophilic so they tend to be quite stable in aqueous solutions, and they preserve the conformation of such substances when such substances are linked to solid phases. At the same time, these linking groups are of such lengths that the solid phases tend not to interfere with binding sites on such substances.

Solid phase conjugates of this invention are of formula II: 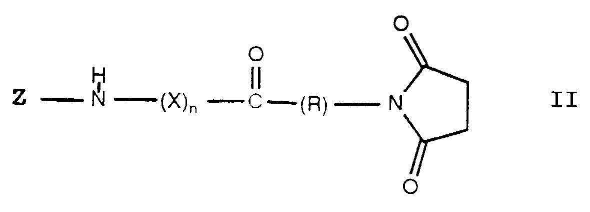
wherein Z is an amine bearing solid phase material; X is an amino acid having from three to ten carbon atoms in a straight chain; n is from one to ten; and R is an alkyl, cycloalkyl, an alkyl-cycloalkyl or phenyl.

The current invention also involves protein-solid phase conjugates of the formula III: 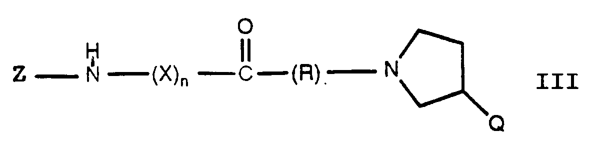
wherein Q is a -SH or a thiol-bearing peptide, polypeptide or protein, or a peptide, polypeptide, or a protein derivatized with sulfhydryl groups; and wherein Z, X, n, and R are as defined previously.

The present invention also includes antibody-enzyme conjugates of formula IV: 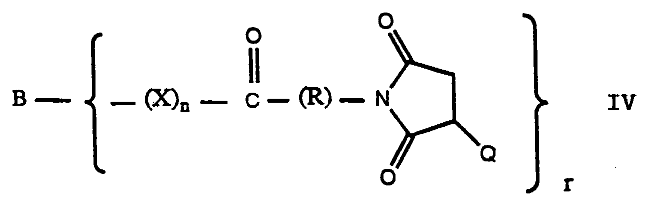
wherein X, n, and R are as defined above; B is an antibody, antibody fragment or enzyme; Q is an antibody or antibody fragment when B is an enzyme, and an enzyme when B is an antibody or antibody fragment; and r is from one to fifty. With this conjugate, many enzyme markers can be attached to a single antibody (or vice versa) to get a large response in an immunoassay.

The present invention also includes immunogens of formula V: 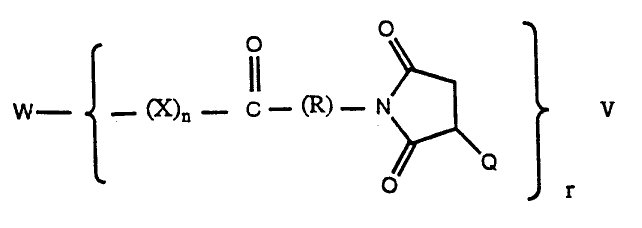
where X, n, and R are as defined above, W is a polyaminoacid, Q is a hapten, and r is 1 to 20.

### Detailed Description of the Invention

The coupling agent of formula I can be used to conjugate proteins to solid phases, enzymes to antibodies, haptens to hapten carriers, or virtually any protein to another protein. Using a coupling agent of formula I in such conjugates produces a conjugate where the normal activity of the antibody, enzyme or protein is retained. It is believed that prior coupling agents, by virtue of their short overall length in comparison with the size of the proteins being conjugated produce conjugates in which the conformational freedom of the individual proteins is compromised. This loss of conformational freedom can lead to diminished activity of either or both proteins in the conjugate resulting in poorer performance in EIAs or poorer stability of the reagent. Thus, many prior coupling agents have limited usefulness.

The long hydrophylic chain of amino acids in the coupling agents of the present invention not only provide better physical separation of the conjugated proteins, but also are solvated well by water in the solution, in contrast to often-used hydrophobic spacer groups in previous coupling reagents. The coupling agents of this invention are thus well suited for use in aqueous solutions.

As indicated above, the present invention also involves conjugates of "amine bearing solid phases." Such solid phases include those polymers, glasses, and natural products which bear amine (either primary, secondary, or tertiary) groups which can be reacted with a maleimide moiety to form a stable covalent bond. A wide variety of solid phases are possible consistent with this definition: commercially available polystyrene aminated particles, amino silica gels, partially hydrolyzed nylon, partially reduced polyacrylamides, partially reduced cyanoacrylates and copolymers containing such polymers. Solid phases containing nitrile groups which can be reduced to yield amine groups produce "amine-bearing solid phases" consistent with this invention.

While it is preferred to use microparticle solid phases, other solid phase configurations are possible: beads, sheets, spheres, filters, and the like. However, one solid phase configuration of particular interest is fiber. Many of the polymers mentioned previously are available in fiberous form. These fibers can be chopped (discontinuous) or continuous, but the latter are preferred. Continuous fibers can be derivitized with the linking groups of this invention, and proteins such as antibodies can be conjugated to the linking groups. The protein-bearing fiber can then be sewn or woven into a solid support such as cloth, a mat, or a woven or non-woven filter media. The fiber can be sewn or woven into distinctive patterns. For example, the fibers can be arranged in a plus (+) sign with the vertical bar having positive controls and the horizontal bar having negative controls, as disclosed in copending U.S application serial number 831,013. Thus, when an assay is performed using the sewn fibers, a plus sign will appear if the sample passed through the media has analyte in it, and a minus (-) (horizontal bar only) will appear if no analyte is in the sample.

Another approach is to attach the linking groups of this invention to the fiber, sew the fiber into an inert backing material (i.e. a material which lacks maleimide groups), and react the protein to the exposed maleimide moieties on the linking groups.

The substituent "Q" is a -SH or thiol-bearing peptide, polypeptide or protein. For convenient reaction with the linking groups of this invention, the peptide, polypeptide or protein to be conjugated to the linking groups bear reactive thiol (mercapto) or sulfhydryl (-SH) groups. It is recognized that mercapto groups contain sulfhydryl groups, but this invention contemplates that peptides, polypeptides or proteins lacking sulfhydryl groups may be artificially derivatized with sulfhydryl groups which are not mercapto groups, mercapto groups being organic compounds bearing sulfhydryl groups. The sulfhydryl group on the peptide, polypeptide or protein reacts with maleimide moiety on the linking groups of this invention to form a covalent bond between the linking groups and the peptide, polypeptide or protein.

The term "alkyl-cycloaklyl" as used herein for "R" includes alkyl groups linked to cycloalkyl ring structures where the alkyl group links the cycloalkyl to the maleimide or the carbonyl groups. The term "alkyl" includes straight or branched alkyl groups, preferably lower groups having from one to six carbon atoms.

The Examples which follow illustrate this invention. Examples 1-8 describe the synthesis of various linker groups of this invention. Examples 9-27 describe the uses of the linker groups to conjugate solid phases to proteins, and proteins to proteins.

### Example 1

### Synthesis of:

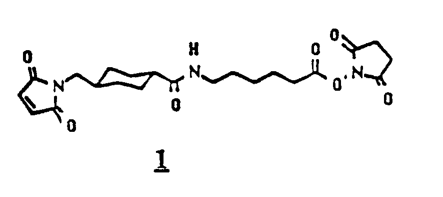

Trans-4-(aminomethyl)-cyclohexanecarboxylic acid was purchased from Aldrich Chemical Co. and converted to N-(4-carboxycyclohexylmethyl) maleimide by the method of Yoshitake et al. (J. Biochem., 101:395-399 (1979)). This material (100 mg) is then dissolved in dry dimethylformamide (DMF) (1.0 ml), 6-aminocaproic acid (39.2 mg; 1.0 eq) is added, and the resulting mixture is stirred overnight at room temperature under nitrogen atmosphere. The following morning, dicyclohexyl-carbodiimide (DCCI) (67.8 mg; 1.1 eq) is added, and the reaction mixture is stirred for an additional six hours. Precipitated dicyclohexylurea (DCU) is removed by filtration, and the resulting DMF solution is evaporated under reduced pressure to give a tacky solid, which is purified by flash chromatography upon silica gel (5% methanol/chloroform) to give compound 1 (71 mg) as a white solid in 53% overall yield. (R=cyclohexylmethyl; n=1; X=6-aminocaproyl).

### Example 2

### Synthesis of:

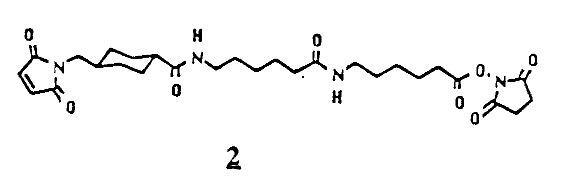

Compound 1 (100 mg; synthesis described in Example 1) is dissolved in dry DMF (1.0 ml), 6-aminocaproic acid (29.3 mg; 1.0 eq) is then added, and the resulting mixture is stirred overnight at room temperature under nitrogen atmosphere. The following morning, DCCI (50.7 mg; 1.1 eq) is added, and the reaction mixture is stirred for an additional six hours. Solid precipitate (DCU) is removed by filtration, and the resulting DMF solution is evaporated under reduced pressure to produce a tacky solid, which is purified by flash chromatography upon silica gel (10% methanol/chloroform to give compound 2 (60 mg) as a white solid in 48% overall yield. (R=cyclohexylmethyl; n=2; X=6-aminocaproyl).

### Example 3

### Synthesis of:

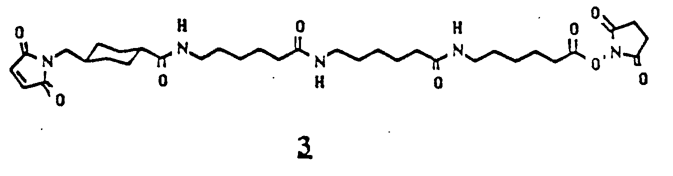

Compound 2 (100 mg; synthesis described in Example 2) is dissolved in dry DMF (2.0 ml), 6-aminocaproic acid (23.4 mg; 1.0 eq) is then added, and the resulting mixture is stirred overnight at room temperature under nitrogen atmosphere. The following morning, DCCI (40.5 mg; 1.1 eq) is added and the reaction mixture is stirred for an additional six hours. Solid precipitate (DCU) is removed by filtration, and the resulting DMF solution is evaporated under reduced pressure to give a tacky solid, which is purified by flash chromatography upon silica gel (10% methanol/chloroform) to give compound 3 (60.0 mg) as a white solid in 50% overall yield. (R=cyclohexylmethyl; n=3; X=6-aminocaproyl).

### Example 4

### Synthesis of:

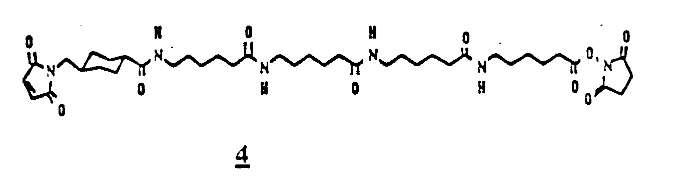

Compound 3 (100 mg; synthesis described in Example 3) is dissolved in dry DMF (10.0 ml), 6-aminocaproic acid (19.5 mg; 1.0 eq) is then added, and the resulting mixture is stirred overnight at room temperature under nitrogen atmosphere. The following morning, DCCI (33.7 mg; 1.1 eq) is added, and the reaction mixture is stirred for an additional six hours. Solid precipitate (DCU) is removed by filtration, and the resulting DMF solution is evaporated under reduced pressure to give a tacky solid, which is purified by flash chromatography upon silica gel (10% methanol/chloroform) to give compound 4 (53 mg) as a white solid in 45% overall yield. (R=cyclohexylmethyl; n=4; X=6-aminocaproyl).

### Example 5

### Synthesis of Extended Length MBS Linkers

### a) Synthesis of:

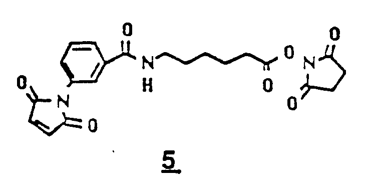

A 0.15 M solution of m-maleimidobenzoyl N-hydroxysuccinimide ester (MBS) (prepared according to the procedure of Kitagawa, et al., J. Biochem., 79:233-236 (1976)) in dry DMF is treated with one equivalent of 6-aminocaproic acid and stirred at room temperature overnight under nitrogen atmosphere. The following morning, 1.0 equivalent of dicyclohexylcarbodiimide (DCCI) is added, and the reaction mixture is stirred for an additional six hours at room temperature. Precipitated dicyclohexylurea (DCU) is then removed by filtration, and DMF is evaporated under high vacuum to give a crude product 5 which is purified by flash chromatography upon a silica gel column. (R=3,5-disubstituted benzene ring; n=1; X=6-aminocaproyl).

### b) General Procedure for Synthesis of Extended Length Active Esters

To synthesize even longer linker arms based on MBS, or any active ester, a DMF solution of a compound containing a N-hydroxysuccinimidyl ester (e.g., compound 5 from Part a) is treated with 1.0 equivalent of 6-aminocaproic acid and stirred at room temperature under nitrogen atmosphere until no more active ester is visible by TLC. 1.1 equivalent DCCI is then added, and the reaction mixture visible by TLC. Precipitated DCU is then removed by filtration, DMF is evaporated under reduced atmosphere to yield a crude residue which is purified by flash chromatography on silica.

This procedure inserts a 6-aminocaproic acid residue into an active ester, and re-synthesizes the active ester. This insertion procedure can be repeated to yield a reagent of desired length, using 6-aminocaproic acid, or other amino acids.

### Example 6

### Synthesis of Extended Length MCS Reagents

### a) Synthesis of:

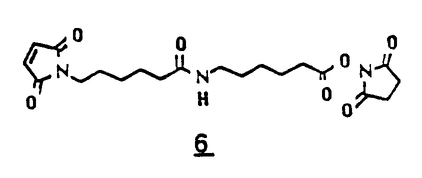

A 0.15 M solution of 6-maleimidocaproic acid N-hydroxysuccinimide ester (MCS) (prepared according to the procedure described by Keller and Rudinger; Helv. Chim Acta, 58:531-541 (1975)) in dry DMF is treated with one equivalent of 6-aminocaproic acid, and allowed to stir overnight at room temperature under a nitrogen atmosphere. The following morning, 1.1 equivalent DCCI is added, and the reaction mixture is stirred for an additional six hours. Precipitated DCU is then removed by filtration, DMF is evaporated under high vacuum to give a crude product 6 which is purified by flash chromatography on silica. Longer analogs of 6 can be made by following the general homologation sequence described in Example 10, Part b. (Formula I; R=a 1,5-disubstituted pentyl group, n=1; X=6-aminocaproyl).

### Example 7

### Synthesis of:

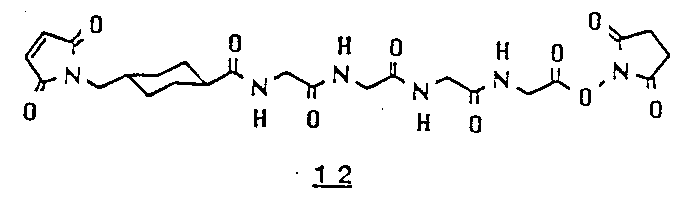

### a) Synthesis of Compound 7

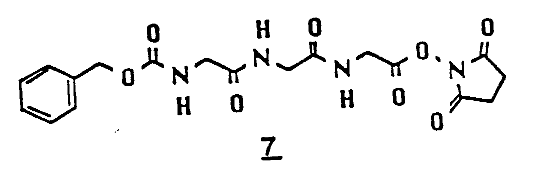

CBZ-triglycine (4.0 g; Bachem Chem. Co.) is dissolved in 50.0 ml dry DMF. N-hydroxysuccinimide (1.42 g; 1.0 eq), and DCCI (2.55 g; 1.0 eq) are added, and the resulting mixture is stirred overnight at room temperature under nitrogen atmosphere. The following morning, precipitated DCU is removed by filtration, and the resulting DMF solution is evaporated under reduced pressure to give a yellow oil. Recrystallization from ethyl acetate/chloroform gives the intermediate compound 7 (3.0 g) in 57% yield.

### b) Synthesis of Compound 8

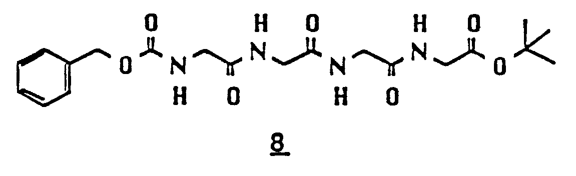

Glycine t-butyl ester hydrochloride (0.54 g; Sigma Chem. Co.) is suspended in dry DMF (25.0 ml). Compound 7 (1.35 g; 1.0 eq) from Part a is then added, along with triethylamine (1.62 g; 5.0 eq). The resulting solution is allowed to stir overnight at room temperature under nitrogen atmosphere. The following morning, solvent is removed under reduced pressure to give a crude product. Recrystallization from ethyl acetate/chloroform gives intermediate compound 8 (0.95 g) in 68% yield.

### c) Synthesis of Compound 9

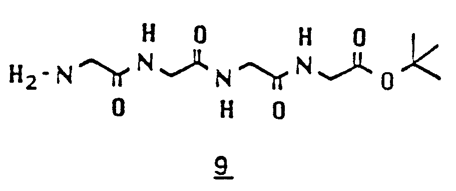

Compound 8 (0.95 g) from Part b) is dissolved in dry methanol (300 ml). Glacial acetic acid (0.45 ml) is then added, and the solution is purged with nitrogen for 15 minutes. Palladium on carbon (1.5 g; palladium content 10%) is then carefully added, while the mixture is stirred. A stream of hydrogen gas is bubbled through the stirring solution for three hours at room temperature. The solution is carefully purged with nitrogen for 15 minutes, then filtered. The filtrate solution is concentrated under reduced pressure to give intermediate compound 9 (700 mg) as the acetate salt.

### d) Synthesis of Compound 10

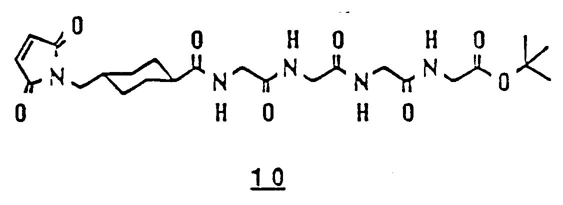

Compound 9 (700 mg: acetate salt) from Part c) is dissolved in dry DMF (25 ml). N-(4-carboxycyclohexylmethyl)maleimide (697 mg) from Example 1 is then added, and the mixture is allowed to stir overnight at room temperature under nitrogen atmosphere. The following morning, DMF is evaporated under reduced pressure to yield a crude product. Recrystallization from ethyl acetate/hexane affords intermediate compound 10 in 22% yield.

### e) Synthesis of Compound 11

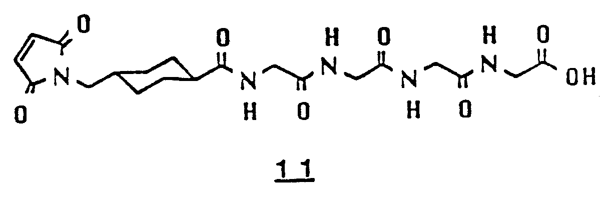

Compound 10 (225 mg) from Part d is suspended in chloroform (1.5 ml). Dry trifluoroacetic acid (1.5 ml) is then added, and the mixture is stirred at room temperature under a nitrogen atmosphere for a period of three hours. Solvent is evaporated under reduced atmosphere to give a crude product. Titration with ethyl acetate gives intermediate compound 11 (127 mg) in 61% yield.

### f) Synthesis of Compound 12

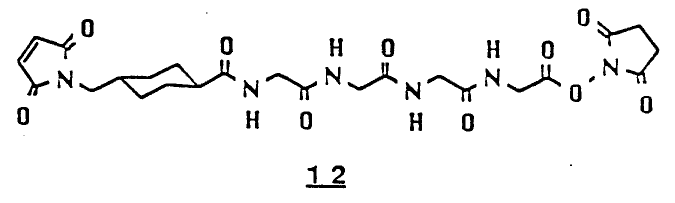

Compound 11 (100 mg) from Part e is dissolved in dry DMF (7.0 ml) along with N-hydroxysuccinimide (37.1 mg; 1.5 eq) and DCCI (221.5 mg; 5.0 eq). The reaction mixture is stirred overnight at room temperature under a nitrogen atmosphere. The following morning, precipitated DCU is removed by filtration, and DMF is evaporated under reduced pressure to give a crude solid. Titration with chloroform gives compound 12 (86 mg) in 60% yield. (R=cyclohexylmethyl; n=4; X=glycyl).

Compound 12 can be used for conjugating proteins (e.g. antibody and enzymes) to solid phases using the procedures outlined in the following Examples.

### Example 8

### Synthesis of m-Maleimidobenzoylcaproamido-N-Hydroxy Succinimide

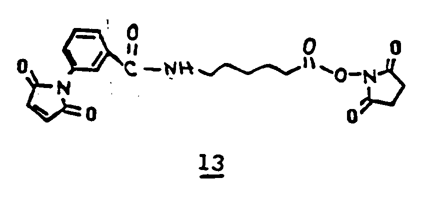

A round bottom flask equipped with a magnetic stirrer is charged with m-maleimidobenzoyl-N-hydroxysuccinimide ester (0.314 g; 0.001 mole) obtained from Pierce Corporation dissolved in DMF (5.0 mL). 6-Aminocaproic acid (0.131 g; 1 equiv.) is added, and the resulting solution is stirred overnight at room temperature under nitrogen. After 18 hours, dicyclohexylcarbodiimide (DCCI; 0.206 g; 1.1 equiv.) is added followed by N-hydroxysuccinimide (0.115 g, 1 equiv.). The reaction solution is stirred for additional eight hours at room temperature under nitrogen. Precipitated dicyclohexylurea (DCU) is removed by filtration, and the resulting DMF solution is evaporated under reduced pressure. The resulting solid is purified by silica gel chromatography (5% methanol in chloroform) to give compound 13 in 50% yield. This compound is treated with aminocaproic acid in a manner identical to the method described in examples 2, 3 and 4 of this application to produce compounds where n is up to ten, and R=phenyl).

### Example 9

### Conjugation of Monoclonal Anti-Alpha Fetoprotein IgG With Calf Intestinal Alkaline Phosphatase Using Compound 3

### a) Derivatization of Enzyme

A solution of calf intestinal alkaline phosphatase (250 µl; 10 mg/ml; Boehringer-Mannheim) is placed in a vial. A solution of compound 3 in DMF (100 µl; 5.0 mM) is added, and the resulting mixture is stirred on a rotary agitator for thirty minutes at room temperature. The crude reaction mixture is purified by chromatography upon a pre-equilibrated Sephadex® G-25 (coarse) column with pH 7.0 phosphate buffer (0.1 M phosphate, 0.1 M NaCl, 1 mM MgCl₂, 0.1 mM ZnCl₂) as eluent.

Fractions from the column are collected, enzyme-containing fractions are pooled, and protein concentration of the pooled fractions is estimated by measuring absorbance at 280 nm. In our hands, the absorbance at 280 nm was found to be approximately equal to protein concentration in mg/ml.

### b) Antibody Derivatization

Monoclonal anti-AFP IgG solution at 6.4 mg/ml is incubated and stirred on a rotary agitator at room temperature with DTT (dithiothreotol; 25 mM concentration in the final reaction solution) for twenty minutes. The solution of partially reduced antibody is then purified by chromatography upon a pre-equilibrated Sephadex® G-25 (coarse) column with pH 7.0 phosphate buffer (0.1 M phosphate, 0.1 M NaCl, 5 mM ERDTA) as eluent.

Fractions from the column are collected, protein-containing fractions are pooled, and protein concentration of the pooled solution is estimated by measuring absorbance at 280 nm. In this instance, absorbance at 280 nm divided by a factor of 1.39 is approximately equal to antibody concentration in mg/ml.

### c) Conjugation of Partially Reduced Antibody With Derivatized Alkaline Phosphatase

The derivatized alkaline phosphatase from Part a is combined with the partially reduced antibody from Part b in a molar ratio of 1.5:1 enzyme to antibody. The mixture is stirred overnight at 2-8°C on a rotary agitator. The following morning, unreacted thiol groups are capped by treatment with an N-ethylmaleimide (NEM) solution (100 µl; 5.0 mM) for a period of one hour at room temperature. The conjugate concentrate thus obtained can be diluted as necessary for use in a sandwich assay or any other assay where a labeled antibody is to be used. (Formula IV; B=calf intestinal alkaline phosphatase; R=cyclohexylmethyl; X=6-aminocaproyl; n=3; Q=monoclonal anti-AFP IgG; and r is from one to five).

### Example 10

### Conjugation of β-Galactosidase to Goat Anti-Rabbit IgG Using Compounds 1, 2, or 3

### a) Antibody Derivatization with Compound 1

Goat anti-rabbit IgG (1.0 mg) is suspended with pH 7.0 phosphate buffer (1.0 ml; 15 mM phosphate; 150 mM NaCl; 1.0 mM EDTA). An aliquot of this solution (425 µl) is then added to a solution of compound 1 in DMF (12.75 µl; 6.0 mM). The mixture is incubated in the dark for sixty minutes at room temperature with occasional mixing. The crude reaction mixture is then dialyzed overnight in the dark at 4°C against pH 7.0 phosphate buffer (2.01; 15 mM phosphate; 150 mM NaCl; 1 mM EDTA). The dialyzed material is recovered by centrifugation, and antibody concentration is estimated by measuring absorbance at 280 nm. In our hands A₂₈₀ divided by a factor of 1.38 is approximately equal to antibody concentration in mg/ml.

### b) Conjugation of Derivatized Antibody With Native β-Galactosidase

The derivatized antibody from Part a and native E. coli β-galactosidase (25 mg/ml in pH 7.0 phosphate buffer) are combined in a molar ratio of 0.6:1 enzyme to antibody. The mixture is allowed to react overnight at room temperature without stirring to yield a concentrated solution of conjugate for use in an immunoassay. (Formula IV; B=goat anti-rabbit IgG; R=cyclohexylmethyl; X=6-aminocaproyl; n=1; Q=β-galactosidase and r=from one to five).

### c) Antibody Derivation with Compound 2

Goat anti-rabbit IgG (1.0 mg) is suspended with pH 7.0 phosphate buffer (1.0 ml; 15 mM phosphate; 150 mM NaCl; 1.0 mM EDTA). An aliquot of this solution (425 µl) is then added to a solution of compound 2 in DMF (12.75 µl; 6.0 mM). The mixture is incubated in the dark for sixty minutes at room temperature with occasional mixing. The crude reaction mixture is then dialyzed overnight in the dark at 4°C against pH 7.0 phosphate buffer (2.0 l ; 15 mM phosphate; 150 mM NaCl; 1 mM EDTA). The dialyzed material is recovered by centrifugation, and antibody concentration is estimated by measuring absorbance at 280 nm. In our hands A₂₈₀ divided by a factor of 1.38 is approximately equal to antibody concentration in mg/ml.

Antibody derivatized with compound 2 can be coupled to native β-galactosidase as described in Part b above. (Formula IV; B=goat anti-rabbit IgG; R=cyclohexylmethyl; X=6-aminocaproyl; n=2; Q=β-galactosidase, and r=from one to five).

### D) Antibody Derivatization with Compound 3

Goat anti-rabbit IgG (1.0 mg) is supended in pH 7.0 phosphate buffer (1.0 ml; 15 mM phosphate; 150 mM NaCl; 1.0 mM EDTA). An aliquot of this solution (425 µl) is then added to a solution of compound 3 in DMF (12.75 µl; 6.0 mM). The mixture is incubated in the dark for sixty minutes at room temperature with occasional mixing. The crude reaction mixture is then dialyzed overnight in the dark at 4°C against pH 7.0 phosphate buffer (2.0 l; 15 mM phosphate; 150 mM NaCl; 1 mM EDTA). The dialysed material is recovered by centrifugation, and antibody concentration is estimated by measuring absorbance at 280 pm. In our hands A₂₈₀ divided by a factor of 1.38 is approximately equal to antibody concentration in mg/ml.

Antibody derivatized with compound 3 can be coupled to native β-galactosidase as described in Part b above. (Formula IV; B=goat anti-rabbit IgG; R=cyclohexylmethyl; X=6-aminocaproyl; n=3; Q=β-galactosidase, and r=from one to five).

### Example 11

### Preparation of Polyclonal anti-TSH (Thyroid Stimulating Hormone)-Alkaline Phosphatase Conjugate Using Compound 1

### a) Derivatization of the Enzyme

Calf intestinal alkaline phosphatase (25 µl; 10 mg/ml) is combined with a solution of compound 1 in DMF (10 µl; 7.8 mM) and reacted for thirty minutes at room temperature. The crude reaction mixture is purified by passage through a mini-gel filtration (G-25 Sephadex®) column while centrifuging, the column having been equilibrated with 0.05 M phosphate buffer at pH 7.0. A purified solution of a linker-derivatized enzyme is obtained.

### b) Antibody Derivatization

A polyclonal anti-TSH IgG solution (25 µl; 5.3 mg/ml) is mixed with a solution of DTT (25 µl; 0.1 M) in pH 7.0 phosphate buffer (0.5 M phosphate). The mixture is allowed to react for thirty minutes at room temperature, then purified in the same manner as the derivatized enzyme in Part a).

### c) Conjugation of Enzyme and Antibody

The products of Parts a and b are combined, incubated overnight at 2-8°C, and a conjugate of anti-TSH with alkaline phosphatase is obtained. This conjugate can be used in sandwich assays for TSH. (Formula IV; B=alkaline phosphatase; R=cyclohexylmethyl; X=6-aminocaproyl; n=1; Q=polyclonal anti-TSH IgG; and r=from one to five).

### Example 12

### Conjugation of Horseradish Peroxidase to Fab' Anti-Hepatitis B Core Antibody Using Compound 3

### a) Derivatization of the Enzyme

A horseradish peroxidase solution (400 µl; 10 mg/ml) in pH 7.0 phosphate buffer (0.1 M phosphate; 0.1 M NaCl) is added to a solution of compound 3 (3.3 mg) in DMF (236 µl). The mixture is stirred for 30 minutes at room temperature, then desalted on a Sephadex® G-25 (coarse) column using the above described buffer. Fractions are collected, enzyme-containing fractions pooled, and concentration of pooled enzyme is estimated by measuring absorbance at 280 nm. In our hands, A₂₈₀ divided by a factor of 0.61 is approximately equal to enzyme concentration in mg/ml.

### b) Derivatization of the Antibody

A solution of Fab' anti-hepatitis B core IgG fragment (3.1 ml; 3.2 mg/ml) is placed in a vial. Phosphate buffer (pH 7.0; 0.5 M phosphate; 0.5 M NaCl; 25 mM EDTA) is added. DTT (23.1 mg) is then added, and the mixture is incubated at room temperature for twenty minutes while stirring.

The crude product is then placed on a G-25 Sephadex® column (coarse) and eluted with the above described pH 7.0 phosphate buffer.

Fractions are collected, protein-containing fractions are pooled, and concentration of the pool is estimated by measuring absorbance at 280 nm. In our hands A₂₈₀ divided by a factor of 1.38 is approximately equal to antibody fragment concentration in mg/ml.

### c) Conjugation of Derivatized Alkaline Phosphatase With Antibody Fragment

Derivatized enzyme from Part a is combined with the antibody fragment from Part b in a 1:1 enzyme to antibody ratio. The mixture is incubated overnight on a rotary agitator at 2-8°C. The following morning, the crude conjugate mixture is passed over a Con A-sepharose column. Unconjugated antibody fragments are washed away, and then conjugate is eluted with a 0.1 M alpha-methyl glucoside solution. Recovered conjugate is dialyzed against buffer and diluted for use in a sandwich assay for hepatitis B viral particles. (Formula IV; B=alkaline phosphatase; R=cyclohexylmethyl; X=6-aminocaproyl; n=3; Q=Fab' anti-hepatitis B core antibody; and r is from one to five).

### Example 13

### Conjugation of Calf Intestinal Alkaline Phosphatase to Bovine Gamma Globulin Specific for E. Coli Cell Wall Antigens Using Compound 2

### a) Enzyme Derivatization with Iminothiolane

To a solution of calf intestinal alkaline phosphatase (2.0 ml; 12.3 mg/ml) in pH 8.0 tris buffer (0.05 M triethanolamine (tris); 10 mM MgCl₂; 0.1 mM ZnCl₂) is added iminothiolane HCl such that final iminothiolane concentration in the reaction mixture is 4.0 mM. The reaction mixture is stirred for one hour at 4°C. Excess glycinamide is then added to quench unreacted iminothiolane. The crude reaction mixture is placed on a TSK 40 column and eluted with the pH 8.0 tris buffer described above. Column fractions are collected, enzyme-containing fractions are pooled, and protein concentration of the pool is estimated by measuring absorbance at 280 nm. In our hands A₂₈₀ divided by a factor of 0.76 is approximately equal to enzyme concentration in mg/ml.

### b) Antibody Derivatization with Compound 2

A solution of bovine gamma globulin (2.0 ml; 8.4 mg/ml; raised against cell wall antigens of E. Coli) in pH 8.0 tris buffer (0.5 M tris; 0.16 M NaCl; 1.0 mM MgCl₂; 0.1 mM ZnCl₂) is treated with a solution of compound 2 (3.36 mg) dissolved in DMF (859 ul) and incubated for one hour at 4°C while the mixture is stirred.

The crude reaction mixture is then placed on a TSK 40 column and eluted with the pH 8.0 tris buffer described above.

Column fractions are collected, protein-containing fractions are pooled, and the protein concentration of the pool is estimated by measuring absorbance at 280 nm. In our hands A₂₈₀ divided by a factor of 1.40 is approximately equal to enzyme concentration in mg/ml.

### c) Conjugaton of Enzyme and Antibody

Derivatized enzyme from Part a is combined with derivatized antibody from Part b in a 1.1:1 (enzyme:antibody) ratio, and stirred at 4°C overnight. The following morning, the crude reaction mixture is added to an equal volume of pH 8.0 tris buffer (0.05 M tris; 1.0 mM MgCl₂; 0.1 mM ZnCl₂; 0.16 M NaCl; 2% bovine serum albumin (BSA); 0.2% sodium azide). β-mercaptoethanol (10 µl) is then added, and the crude conjugate concentrate is stored in this form until needed. A 1:1000 dilution of this conjugate concentrate with an appropriate diluent gives about a 0.5 µg/ml solution of conjugate which can be used in a sandwich-type assay for various microorganisms. (Formula IV; B=bovine gamma globulin; R=cyclohexylmethyl; X=6-aminocaproyl; n=2; Q=calf intestinal alkaline phosphatase; and r is from one to five).

### Example 14

### Conjugation of Monoclonal anti-Carcinoembryonic Antigen (CEA) IgG to Calf Intestinal Alkaline Phosphatase Using Compound 3

### a) Enzyme Derivatization

A solution of calf intestinal alkaline phosphatase (500 µl; 10.7 mg/ml) is mixed with a solution of compound 3 in dry DMF (200 µl; 5.0 mM) and stirred at room temperature for 30 minutes. The crude reaction mixture is then placed on a pre-equilibrated Sephadex® G-25 column, and eluted with pH 7.0 phosphate buffer (0.1 M phosphate; 0.1 M NaCl 1 mM MgCl₂; 0.1 mM ZnCl₂).

Fractions from the column are collected, enzyme-containing fractions are pooled, and the enzyme concentration of the pooled fractions is estimated by measuring absorbance at 280 nm. In our hands, the absorbance at 280 nm was found to be approximately equal to protein concentration in mg/ml.

### b) Antibody Derivatization

A solution of monoclonal anti-CEA IgG at (315 µl; 9.5 mg/ml) is treated with 500 molar equivalents of a solution of iminothiolane hydrochloride in pH 8.0 phosphate buffer (0.033 mg/ml iminothiolane; 0.1 M phosphate; 0.1 M NaCl; 5 mM EDTA). The reaction mixture is stirred at room temperature for 30 minutes, then placed on a pre-equilibrated Sephadex® G-25 column, and eluted with the pH 8.0 phosphate buffer described above.

Fractions from the column are collected, antibody containing fractions are poooled, and protein concentration of the antibody pool is estimated by measuring absorbance at 280 nm. In our hands, A₂₈₀ divided by a factor of 1.39 is approximately equal to antibody concentration in mg/ml.

### c) Conjugation of Enzyme and Antibody

Derivatized enzyme from Part a and derivatized antibody from Part b are combined in a 1:1 molar ratio, and stirred overnight at 2-8°C. The following morning, the conjugate is diluted to approximately 1 µg/ml concentration with an appropriate diluent, and used directly in a sandwich-type assay for carcinoembryonic antigen (CEA). (Formula IV; B=calf intestinal alkaline phosphatase; R=cyclohexylmethyl; X=6-aminocaproyl; n=3; Q=monoclonal anti-CEA IgG; and r is from one to five).

### Example 15

### Synthesis of Immunogen

### a) Functionalization of BSA with Compound 1

BSA (50.0 mg) is dissolved in 5.0 ml pH 6.6 phosphate buffer (0.1 M phosphate). To this solution is added a solution of compound 1 (5.0 mg) in DMF (500 µl). The reaction mixture is stirred overnight at room temperature. The following morning, the crude reaction mixture is applied to a Sephadex® G-25 column and eluted with water.

Fractions from the column are collected, protein-containing fractions are pooled and lyophilized to give 56 mg of derivatized BSA. Titration of maleimide groups indicated incorporation of about nine maleimide groups per BSA.

### b) Synthesis of the Immunogen

A synthetic peptide (10 mg) (MW 3,300) corresponding to amino acids 38 through 71 of the beta subunit of thyroid stimulating hormone (TSH) is dissolved in a mixture of DMF (1.0 ml) and pH 6.6 phosphate buffer (1.0 ml; 0.1 M phosphate). This material shows the presence of one sulfyhdryl group per peptide as determined by Ellman's reagent titration.

To the solution of β-TSH peptide was added modified BSA (1.5 mg) from Part a. The solution is stirred overnight at room temperature in a capped vial. Purification of the crude reaction mixture by chromatography on a Sephadex® G-25 column with water as eluent, followed by lyophilization of the recovered conjugate yields 18 mg of material which after analysis showed incorporation of four β-TSH peptides per BSA. (Formula V; W=BSA; R=cyclohexylmethyl; X=6-aminocaproyl; n=1; Q=TSH subunit peptide; and r=4).

### Example 16

### Preparation of Monoclonal anti-CA-125 IgG Derivatized Microparticles using a 30 Atom Linkage

### a) Pretreatment of Amine Microparticles

Amine microparticles (Seradyn®; 0.164 µm; 2.5% solids; 1 ml) are mixed with 0.5 g Biorex® ion exchange resin (Biorex® MSZ501D; 297-841 µm diameter (20-50 mesh); catalog #142-7425). The mixture is rotated end-over-end for one hour at room temperature, then vacuum filtered through a course sintered glass funnel with washing. The filtrate is collected and centrifuged at 15,000 rpm for 30 minutes. Supernatant is discarded, and the microparticle pellet is resuspended in distilled water with vortexing, and adjusted to 2.5% solids by addition of distilled water.

### b) Derivatization of the Particles

Resuspended, pre-treated particles from part a at 2.5% solids are mixed with an equal volume of compound 3 (Example 3) solution (2 mg/ml in DMF), and reacted at room temperature for one hour with end-over-end rotation. The reaction mixture is then diluted ten-fold with phosphate-buffered saline "PBS" (pH 7.2), and centrifuged at 15,000 rpm for 30 minutes. The resulting supernatant is discarded, and the microparticle pellet is resuspended with phosphate-buffered saline. The centrifugation-resuspension sequence is repeated twice, the solution is again centrifuged, supernatant is discarded, and the pellet is resuspended to a concentration of 2.5% solids with Tris buffer (0.05 M tris; 0.1 M NaCl, pH 8.0).

### c) Preparation of the Antibody

A solution of monoclonal anti-CA-125 IgG (7.4 mg/ml; in phosphate buffered saline) is incubated with DTT (dithiothreitol; 25 mM in final reaction mixture) for twenty minutes at room temperature with stirring on a rotary agitator. The solution of partially reduced antibody is then desalted by chromatography upon a pre-equillibrated Sephadex® G-25 (coarse) column with pH 7.0 phosphate buffer (0.1 M phosphate; 0.1 M NaCl, 5 mM EDTA) as eluent. Fractions are collected, protein-containing fractions are pooled, and the protein concentration of the pooled solution is estimated by measuring absorbance at 280 nm.

### d) Reaction of Maleimide-Derivatized Microparticles with Partially Reduced IgG

The partially reduced antibody from part c (1 ml; 1 mg/ml) is combined with the maleimide-derivatized microparticles from part b (1 ml; 2.5% solids). The mixture is rotated end-over-end at room temperature overnight. The following morning, the reaction mixture is diluted ten-fold with wash buffer (0.01 M phosphate; pH 7.2; 1% Tween®), then centrifuged at 15,000 rpm for 30 minutes. The resulting supernatant is discarded, the microparticle pellet is washed twice (vortexing in 1 ml wash buffer; diluting tenfold with wash buffer, then centrifuging at 15,000 rpm for 30 minutes followed by supernatant removal). The washed pellet is resuspended to a final concentration of 0.125% solids in storage buffer (0.01 M Tris; pH 8.1; 0.1 M NaCl; 0.1% sodium azide; 13.6% sucrose). The final microparticle suspension is first passed through a 23, then a 25 gauge needle. The resulting microparticle conjugate has the anti-CA-125 IgG antibody conjugated to the microparticle with a 30 atom linker arm from Example 3. Microparticles in storage buffer are then stored until future use in an immunoassay for the detection of CA-125 antigen.

### Example 17

### Preparation of Monoclonal anti-CA-125 IgG Derivatized Microparticles using a 23 Atom Linkage

The procedure of Example 16 is repeated using the 23 atom linker group (Compound 2) from Example 2 instead of the 30 atom group in Example 16.

### Example 18

### Preparation of Monoclonal anti-CA-125 IgG Derivatized Microparticles using a 16 Atom Linkage

The procedure of Example 16 is repeated using the 16 atom linker group (Compound 1) from Example 1 instead of the 30 atom group in Example 16.

### Example 19

### Preparation of Polyclonal anti-CA-125 IgG Derivatized Microparticles using a 30 Atom Linkage

The procedure of Example 16 is repeated using the a polyclonal anti-CA-125 IgG antibody instead of the monoclonal antibody of Example 16. Polyclonal anti-CA-125 antibody was obtained by immunizing sheep subcutaneously and intramuscularly with 50,000 units of CA-125 antigen in Freund's adjuvant. All subsequent boosts were done every two weeks using 50,000 units of CA-125 antigen in Freund's incomplete adjuvant.

### Example 20

### Preparation of Monoclonal anti-PAP IgG Derivatized Microparticles using a 30 Atom Linkage

### a) Preparation of Reduced anti-PAP antibody

Dithiothreitol (DTT; 1.93 mg) is placed in a reaction vial. Mouse monoclonal anti-PAP antibody (0.5 ml; 6.98 mg/ml) is then added, and the mixture is incubated for 20 minutes at room temperature. The reaction mixture is then applied to a pre-equilibrated Sephadex® G-25 column (coarse 1 x 20 cm) and eluted with pH 7.2 phosphate buffer (0.2 M phosphate; 0.1 M NaCl; 5.0 mM EDTA). Fractions are collected, absorbance at 280 pm is measured. Protein-containing fractions are combined, the pool is diluted ten-fold with chromatography buffer, and protein concentration of the resulting diluted pool of activated antibody is estimated by measuring absorbance at 280 nm.

### d) Reaction of Derivatized Microparticles with Reduced Anti-PAP Antibody

Reduced antibody from part a (0.5 mg (e.g. 336 µl at 1.49 mg/ml)) is placed in a reaction vial. Derivatized microparticle solution (0.5 ml) from Example 16 part b is then added, and the mixture is incubated overnight at room temperature on a rotary agitator. The following morning, the reaction mixture is centrifuged (20 minutes at 12,000 rpm), supernatant is removed, and absorbance at 280 nm is measured. The antibody-derivatized microparticles are then resuspended in PBS-Tween® buffer (0.1 g Tween® in 100 ml PBS; 1.0 ml). The mixture is again centrifuged (20 minutes at 12,000 rpm), supernatant is discarded, and 5.0 ml storage buffer (0.05 M Tris; 0.1 M NaCl; 0.1% sodium aside; 13.6% sucrose; pH 8.1) is then added. The microparticles are passed first through a 23 gauge needle, then a 25 gauge needle. The resulting microparticle suspension is then transferred to a 10 ml plastic screw-cap vial for storage until use in an enzyme immunoassay for the detection of PAP antigen.

### Example 21

### Covalent Attachment of B-12 Intrinsic Factor to Aminated Polystyrene Microparticles

A 10% solution of 100 µl of aminated polystyrene microparticles (0.164 µm, purchased from Seradyn) is placed in a reaction vial. A solution of Compound 2 in DMF (9 µl; 1.21 mM) is added with a solution of B-12 intrinsic factor (210 µl; 0.1537 mg/ml). The reaction mixture is rotated end-over-end overnight at room temperature. The following morning, the reaction mixture is centrifuged (30 minutes; 13,000 rpm). Supernatant is discarded, the remaining solid is resuspended in distilled water, and the centrifuging-resuspension step is repeated.

The particles are then suspended in 750 µl of storage buffer and used in an assay for the detection of B-12. The product produced is a B-12 intrinsic factor/microparticle conjugate linked with a 23 atom linker of Example 2

### Example 22

### Covalent Attachment of Recombinant Hepatitis B Core Antigen to Aminated Polystyrene Microparticles Using Compound 3

### a) Derivitization of Microparticles with Compound 3

Amino microparticles (Polyscience; 0.5 µm) are placed in a reaction vial. A solution of Compound 3 (Example 3) in DMF is then added, and the mixture is treated as described in Example 16 part b.

### b) Reaction of Recombinant Core Antigen With Derivatized Microparticles

Recombinant hepatitis B core antigen is placed in a vial. Derivatized microparticles (from part a) are then added, and the mixture is treated as decribed in Example 6 part d to yield microparticles conjugated to the antigen with a 30 atom linker which can be used in assays for detection of hepatitis B.

### Example 23

### Preparation of E. coli β-Galactosidase Derivatized Microparcicles With A 30 Atom Linkage

E. coli β-galactosidase (1 ml; 1 mg/ml) in pH 7.0 phosphate buffered saline (0.1M phosphate; 0.1M NaCl) is added to maleimide derivatized microparticles from Example 16, part b. The reaction mixture is rotated end-over-end overnight at room temperature. The following morning, the reaction mixture is centrifuged at 15,000 rpm for 30 minutes. The resulting supernatant is discarded, and the microparticle pellet is resuspended to 2.5% solids with pH 7.0 phosphate buffered saline (0.1M phosphate; 0.1M NaCl). The centrifugation, decanting, resuspension sequence is repeated twice. The microparticle pellet is finally resuspended in storage buffer (0.1M tris; pH 7.0; 0.1M NaCl; 0.1% sodium azide; 13.6% sucrose). The resulting microparticle suspension is passed through first a 23 then a 25 gauge needle. The resulting microparticle conjugated to amino microparticle with a 30 atom linkage. Enzyme-derivatized microparticles in storage buffer are then stored until future use.

### Example 24

### Preparation of Calf Intestinal Alkaline Phosphatase Derivatized Microparticles Using a 30 Atom Linkage

### a) Thiolation of the Enzyme

Calf intestinal alkaline phosphatase (0.5 ml; 10 mg/ml) in pH 8.0 Tris buffer (0.05M Tris; 10 mM MgCl₂; 0.1 mM ZnCl₂) is placed in a reaction vial. Iminothiolane hydrochloride is then added to a concentration of 4.0 mM. The mixture is stirred for 30 minutes at room temperature, then desalted on a Sephadex® G-25 (coarse) column with phosphate buffered Saline (0.1M phosphate; 0.1M NaCl; 10nM MgCl₂, 0.1 M ZnCl₂; pH 7.0) as eluent. Fractions are collected, proteins containing fractions are pooled, and protein concentration of the pooled solution of thiolated enzyme is estimated by measuring absorbance at 2870 nm.

### b) Reaction of Thiolated Enzyme with Maleimide Derivatized Microparticles

Thiolated enzyme from part a (1 ml; 1 mg/ml) is combined with the maleimide-derivatized microparticles from Example 16, part b (1 ml; 2.5% solids). The reaction mixture is rotated end-over-end overnight at room temperature, then treated as described in Example 16, part d to provide a suspension of microparticles covalently derivatized with calf intestinal alkaline phosphatase.

### Example 25

### Preparation of Monoclonal Anti-CA-125 IgG Derivatized Microparticles

### a) Preparation of Thiolated Microparticles

Resuspended, pretreated amine microparticles from Example 16, part a (1 ml; 2.5% solids) are mixed with iminothiolane HCl to achieve a final iminothiolane concentration of 50 mM. The reaction mixture is stirred at room temperature for one hour, then treated as described in Example 16, part b.

### b) Derivatization of the Antibody

A solution of monoclonal anti-CA-125/gG (7.4 mg/ml) in phosphate-buffered saline is incubated with 30 molar equivalents of a DMF solution of Compound 3 (5.0 mM). The reaction mixture is stirred for 30 minutes at room temperature, then desalted on a Sephadex® G-25 (coarse) column with pH 7.0 phospahate buffer (0.1M phosphate; 0.1M NaCl) as eluent. Fractions are collected, protein-containing fractions are pooled, and protein concentration of the pooled solution is estimated by measuring absorbance at 280 nm.

### c) Reaction of Thiolated Microparticles with Maleimide Derivatized Antibodies

Thiolated microparticles from part a (1 ml; 2.5% solid) are mixed with maleimide derivatized antibodies from part b (1 ml; 1 mg/ml). The reaction mixture is rotated end-over-end overnight at room temperature. The following morning, the antibody-derivatized microparticles are treated as described in Example 16, part d to produce a microparticle/IgG conjugate which can be used in an assay to detect CA-125 antigen.

### Example 26

### Preparation of Monoclonal anti-CA-125 IgG Derivatized Nylon Fibers

### a) Pretreatment of Nylon Fibers

Nylon monofilement fishing line (Berkley, 15 cm (6 inches), 0.9 kg (2 lb.) test) is incubated and shaken for 30 minutes at room temperature with 3N HCl (10 ml). The fiber is then washed twice with 20 ml distilled water. The washed, partially hydrolized fiber is stored until further use in distilled water.

### b) Maleimide Derivitization of Partially Hydralyzed Nylon Fiber

A 5 cm (two inch) section of partially hydrolized nylon fiber from part a is cut into 3 mm (1/8 inch) pieces. The 3 mm (1/8 inch) nylon lengths are placed in a reaction vial along with a DMF solution of Compound 3 (1.0 ml; 5.0 mM). The reaction mixture is shaken vigorously for two hours, then filtered through a course sintered glass funnel. The maleimide-derivatized nylon fibers are washed several times with distilled water, then stored in distilled water until further use.

### c) Reaction of Maleimide Derivatized Nylon Fibers with Partially Reduced anti-CA-125 IgG

Partially reduced monoclonal anti-CA-125 IgG from Example 16, part c (1 ml; 1 mg/ml) is added to the maleimide derivatized nylon fibers from part b. The reaction mixture is incubated overnight at room temperature with end-over-end rotation. The following morning, the reaction mixture is filtered through a coarse sintered glass funnel, the antibody derivatized fibers are washed several times with wash buffer (0.1M phosphate; 0.1M Null; pH 7.0). The resulting fibers contain covalently attached monoclonal anti CA-125 IgG via a 30 atom spacer group. The resulting fibers are stored in fiber storage buffer (0.1M, phosphate; 0.1M NaCl; 1% BSA; 0.1% sodium azide) for use in an assay to detect CA-125 antigen.

### Example 27

### Preparationof Monoclonal anti CA-125 IgG Derivatized Wool Fibers

### a) Partial Reduction of Wool Thread

Wool thread is cut into 13 mm (1/2 inch) pieces. A piece of thread is then immersed in 1 ml 5mM DTT solution and shaken vigorously for one hour. The reaction mixture is then filtered through a coarse sintered glass funnel, and washed five times with a buffer (pH 7.0; 0.1M phosphate; 0.1M NaCl; 5mM EDTA).

### b) Reaction of Maleimide Derivatized Antibody With Partially Reduced Wool Thread

Partially reduced wool thread from part a is placed in a reaction vial. Maleimide-derivatized monoclonal anti CA-125 IgG from Example 12, part b (1 ml; 1 mg/ml) is then added, and the mixture is rotated end-over-end overnight at room temperature. The following morning, the reaction mixture is filtered through a coarse sintered glass funnel and washed five times with wash buffer (0.1M phosphate; 0.1M NaCl; pH 7.0). The resulting fiber contains covalently attached monoclonal anti CA-125 IgG via a 30 atom space group. The resulting fibers are stored in fiber storage buffer (0.1M phosphate; 0.1M NaCl; 1% BSA; 0.1% sodium azide) for use in an assay to detect CA-125 antigen.

## Claims (Claims for the following Contracting State(s): AT, BE, CH, DE, FR, GB, GR, IT, LI, LU, NL, SE)

1. A heterobifunctional reagent comprising: 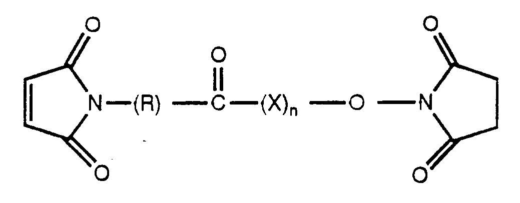 wherein X is an amino acid having from three to ten carbon atoms in a straight chain;
R is an alkyl of one to six carbon atoms, cycloalkyl, alkyl-cycloalkyl wherein said alkyl has from one to six carbon atoms, or phenyl; and
n is from one to ten.

2. The reagent of Claim 1 wherein R is cyclohexylmethyl.

3. The reagent of Claim 1 wherein n is from two to five.

4. The reagent of Claim 3 wherein n is three.

5. The reagent of Claim 1 wherein (X)ₙ is a polyamide formed from repeated units of 6-aminocaproic acid.

6. A conjugate for use in a diagnostic assay, comprising 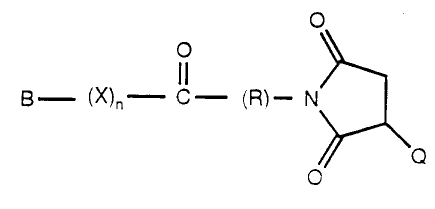 wherein B is an enzyme label, an antibody, or an antibody fragment;
Q is a -SH or thiol-bearing peptide, polypeptide, or protein, or a peptide, polypeptide, or protein derivatized with sulfhydryl groups, provided that when B is an antibody or antibody fragment, Q is an enzyme label and Q is an antibody or antibody fragment when B is an enzyme label;
X is an amino acid having from three to ten carbon atoms in a straight chain;
R is an alkyl of one to six carbon atoms, cycloalkyl, alkyl-cycloalkyl wherein said alkyl has from one to six carbon atoms, or phenyl; and
n is from one to ten.

7. The conjugate of Claim 6 wherein n is from two to five.

8. The conjugate of Claim 6 wherein (X)ₙ is a polyamide formed from repeated units of 6-aminocaproic acid.

9. The conjugate of Claim 6 wherein Q is an enzyme.

10. A conjugate for use in making an immunogen, comprising: 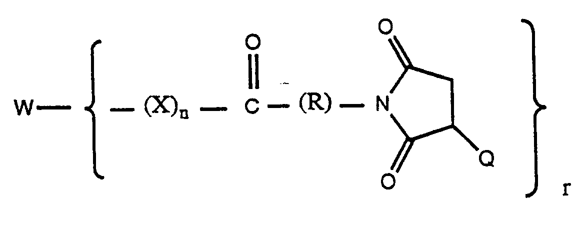 wherein X is an amino acid having from three to ten carbon atoms in a straight chain;
R is an alkyl of one to six carbon atoms, cycloalkyl, alkyl-cycloalkyl wherein said alkyl has from one to six carbon atoms, or phenyl;
n is from one to ten;
Q is a hapten selected from a -SH or thiol-bearing peptide, polypeptide, or protein, or a peptide, polypeptide, or protein derivatized with sulfhydryl groups;
W is a polyaminoacid; and
r is from 1 to 20.

11. A conjugate for use in a diagnostic assay, comprising: 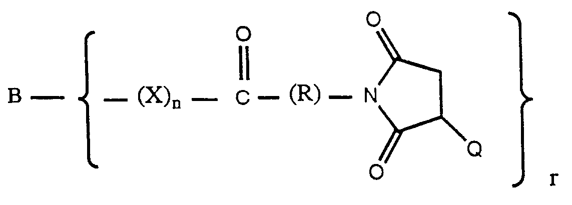 wherein X is an amino acid having from three to ten carbon atoms in a straight chain;
R is an alkyl of one to six carbon atoms, cycloalkyl, alkyl-cycloalkyl wherein said alkyl has from one to six carbon atoms, or phenyl;
n is from one to ten;
B is an enzyme, antibody or antibody fragment;
Q is a -SH or thiol-bearing peptide, polypeptide, or protein, or a peptide, polypeptide, or protein derivatized with sulfhydryl groups, provided that Q is an antibody or antibody fragment when B is an enzyme, and when B is an antibody or antibody fragment, Q is an enzyme; and
r is from two to fifty.

12. The conjugate of Claim 11 wherein B is an enzyme.

13. A solid phase conjugate comprising 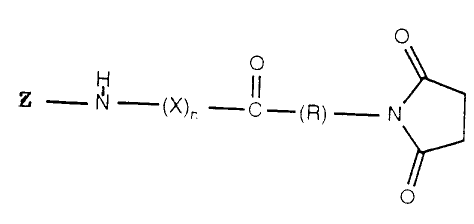 wherein Z is an amine bearing solid phase material; X is an amino acid having from three to ten carbon atoms in a straight chain; n is from one to ten; and R is an alkyl of one to six carbon atoms, cycloalkyl, alkyl-cycloalkyl wherein said alkyl has from one to six carbon atoms, or phenyl.

14. The solid phase conjugate of claim 13 wherein R is cyclohexylmethyl.

15. A conjugate of the formula 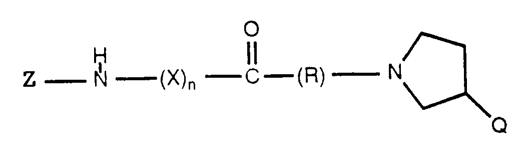 wherein Q is a -SH or thiol-bearing peptide, polypeptide, or protein, or a peptide, polypeptide, or protein derivatized with sulfhydryl groups; Z is an amine bearing solid phase material; X is an amino acid having from three to ten carbon atoms in a straight chain; n is from one to ten; and R is an alkyl of one to six carbon atoms, cycloalkyl, alkyl-cycloalkyl wherein said alkyl has from one to six carbon atoms, or phenyl.

16. The conjugate of Claim 15 wherein Z is an amine bearing microparticle.

## Claims (Claims for the following Contracting State(s): ES)

1. A process for preparing a heterobifunctional reagent comprising a compound of the formula: 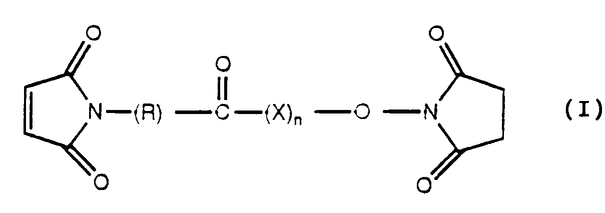 wherein X is an amino acid having from three to ten carbon atoms in a straight chain;
R is an alkyl of one to six carbon atoms, cycloalkyl, alkyl-cycloalkyl wherein said alkyl has from one to six carbon atoms, or phenyl; and
n is from one to ten;
said process comprising reacting a compound of the formula: 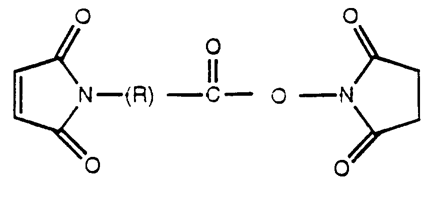 or the corresponding acid thereof with said amino acid X followed by activation and substitution of the terminal acid of the resulting compound to form an active ester of the formula (I) wherein n is one, wherein starting from the latter compound the steps of inserting an amino acid X and synthesizing an active ester may be repeated from one to nine times to produce a compound (I) wherein n is two to ten, respectively.

2. The process of Claim 1 wherein R is cyclohexylmethyl.

3. The process of Claim 1 wherein, starting from the compound of formula (I) wherein n is one, the steps of inserting an amino acid X and synthesizing an active ester are repeated one to four times to produce a compound (I) wherein n is from two to five, respectively.

4. The process of Claim 1 wherein, starting from the compound of formula (I) wherein n is one, the steps of inserting an amino acid X and synthesizing an active ester are repeated twice to produce a compound (I) wherein n is three.

5. The process of Claims 2 through 4 wherein X is 6-aminocaproic acid.

6. A process for preparing a conjugate for use in a diagnostic assay comprising coupling an enzyme label, antibody, or antibody fragment to a compound according to the following formula: 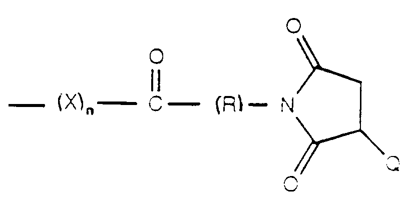 wherein
Q is a -SH or thiol-bearing peptide, polypeptide, or protein, or a peptide, polypeptide, or protein derivatized with sulfhydryl groups, provided that when said compound is coupled to an antibody or antibody fragment, Q is an enzyme label and Q is an antibody or antibody fragment when said compound is coupled to an enzyme label;
X is an amino acid having from three to ten carbon atoms in a straight chain;
R is an alkyl of one to six carbon atoms, cycloalkyl, alkyl-cycloalkyl wherein said alkyl has from one to six carbon atoms, or phenyl; and
n is from one to ten.

7. A process for preparing an immunogen comprising coupling a polyamino acid to a compound according to the following formula: 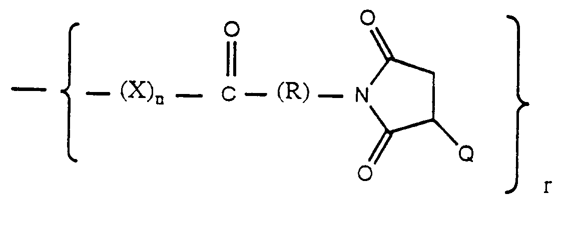 wherein
X is an amino acid having from three to ten carbon atoms in a straight chain;
R is an alkyl of one to six carbon atoms, cycloalkyl, alkyl-cycloalkyl wherein said alkyl has from one to six carbon atoms, or phenyl;
n is from one to ten;
r is from 1 to 20; and
Q is a hapten selected from a -SH or thiol-bearing peptide, polypeptide, or protein, or a peptide, polypeptide, or protein derivatized with sulfhydryl groups;

8. A process for preparing a conjugate for use in a diagnostic assay comprising coupling an enzyme, antibody, or antibody fragment to a compound according to the following formula: 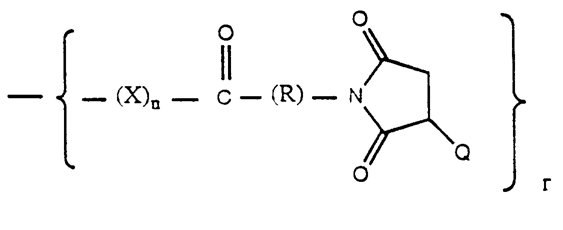 wherein
X is an amino acid having from three to ten carbon atoms in a straight chain;
R is an alkyl of one to six carbon atoms, cycloalkyl, alkyl-cycloalkyl wherein said alkyl has from one to six carbon atoms, or phenyl;
n is from one to ten;
r is from two to fifty; and
Q is a -SH or thiol-bearing peptide, polypeptide, or protein, or a peptide, polypeptide, or protein derivatized with sulfhydryl groups, provided that Q is an antibody or antibody fragment when said compound is coupled to an enzyme, and when said compound is coupled to an antibody or antibody fragment, Q is an enzyme.

9. A process for preparing a solid phase conjugate comprising coupling an amine-bearing solid phase material to a compound according to the following formula: 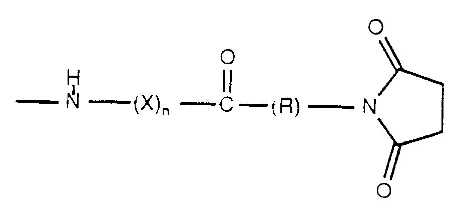 wherein
X is an amino acid having from three to ten carbon atoms in a straight chain;
n is from one to ten; and
R is an alkyl of one to six carbon atoms, cycloalkyl, alkyl-cycloalkyl wherein said alkyl has from one to six carbon atoms, or phenyl.

10. A process for preparing a solid phase conjugate comprising coupling an amine-bearing solid phase material to a compound according to the following formula: 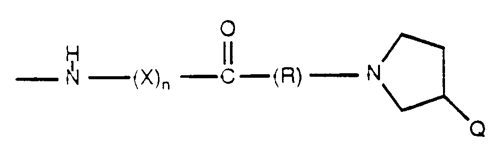 wherein
Q is a -SH or thiol-bearing peptide, polypeptide, or protein, or a peptide, polypeptide, or protein derivatized with sulfhydryl groups;
X is an amino acid having from three to ten carbon atoms in a straight chain;
n is from one to ten; and
R is an alkyl of one to six carbon atoms, cycloalkyl, alkyl-cycloalkyl wherein said alkyl has from one to six carbon atoms, or phenyl.

## Patentansprüche (Patentansprüche für folgende(n) Vertragsstaat(en): AT, BE, CH, DE, FR, GB, GR, IT, LI, LU, NL, SE)

1. Ein heterobifunktionelles Reagens umfassend: 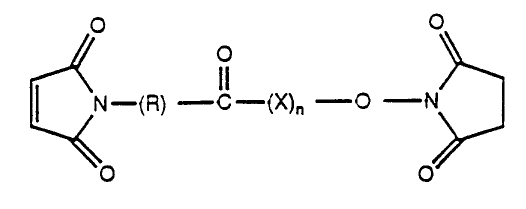 worin X eine Aminosäure mit drei bis zehn Kohlenstoffatomen in einer geraden Kette ist;
R entweder ein Alkyl mit einem bis sechs Kohlenstoffatomen, Cycloalkyl, Alkyl-cycloalkyl, worin das Alkyl eins bis sechs Kohlenstoffatome enthält, oder Phenyl ist; und
n gleich eins bis zehn ist.

2. Das Reagens nach Anspruch 1, worin R Cyclohexylmethyl ist.

3. Das Reagens nach Anspruch 1, worin n gleich zwei bis fünf ist.

4. Das Reagens nach Anspruch 1, worin n gleich drei ist.

5. Das Reagens nach Anspruch 1, worin (X)ₙ ein Polyamid ist, das aus wiederholten 6-Aminocapronsäureeinheiten gebildet wird.

6. Ein Konjugat für die Verwendung in einem Diagnoseassay, umfassend 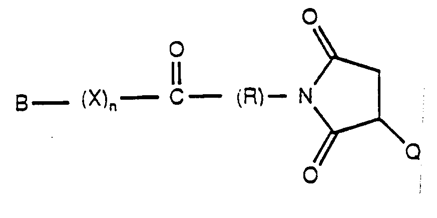 worin B ein Enzymmarkierer, ein Antikörper oder ein Antikörperfragment ist;
Q ein -SH oder thioltragendes Peptid, Polypeptid oder Protein, oder ein Peptid, Polypeptid oder Protein ist, das mit Sulfhydrylgruppen derivatisiert worden ist, vorausgesetzt daß, wenn B ein Antikörper oder Antikörperfragment ist, Q ein Enzymmarkierer ist, und daß, wenn B ein Enzymmarkierer ist, Q ein Antikörper oder Antikörperfragment ist;
X eine Aminosäure mit drei bis zehn Kohlenstoffatomen in einer geraden Kette ist;
R entweder ein Alkyl mit eins bis sechs Kohlenstoffatomen, Cycloalkyl, Alkyl-cycloalkyl, worin das Alkyl eins bis sechs Kohlenstoffatome enthält, oder Phenyl ist; und
n gleich eins bis zehn ist.

7. Das Konjugat nach Anspruch 6, worin n gleich zwei bis fünf ist.

8. Das Konjugat nach Anspruch 6, worin (X)ₙ ein Polyamid ist, das aus wiederholten 6-Aminocapronsäureeinheiten gebildet wird.

9. Das Konjugat nach Anspruch 6, worin Q ein Enzym ist.

10. Ein Konjugat zur Verwendung für die Darstellung eines Immunogens, umfassend 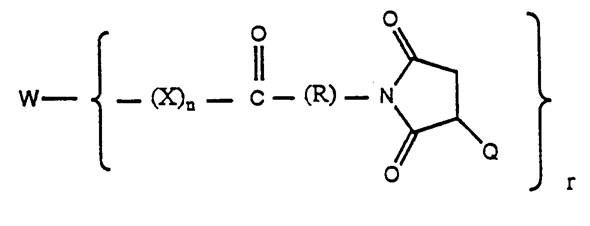 worin X eine Aminosäure mit drei bis zehn Kohlenstoffatomen in einer geraden Kette ist;
R entweder ein Alkyl mit eins bis sechs Kohlenstoffatomen, Cycloalkyl, Alkyl-cycloalkyl, worin das Alkyl eins bis sechs Kohlenstoffatome enthält, oder Phenyl ist;
n gleich eins bis zehn ist;
Q ein Hapten, gewählt aus einem -SH oder thioltragenden Peptid, Polypeptid oder Protein, oder ein Peptid, Polypeptid oder Protein ist, das mit Sulfhydrylgruppen derivatisiert worden ist;
W eine Polyaminosäure ist; und
r gleich 1 bis 20 ist.

11. Ein Konjugat für die Verwendung in einem Diagnoseassay, umfassend: 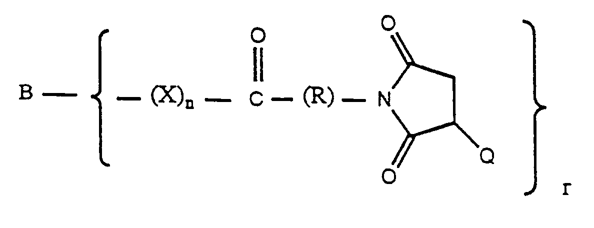 worin X eine Aminosäure mit drei bis zehn Kohlenstoffatomen in einer geraden Kette ist;
R entweder ein Alkyl mit eins bis sechs Kohlenstoffatomen, Cycloalkyl, Alkyl-cycloalkyl, worin das Alkyl eins bis sechs Kohlenstoffatome enthält, oder Phenyl ist;
n gleich eins bis zehn ist;
B ein Enzym, Antikörper oder Antikörperfragment ist;
Q ein -SH oder thioltragendes Peptid, Polypeptid oder Protein, oder ein Peptid, Polypeptid oder Protein ist, das mit Sulfhydrylgruppen derivatisiert worden ist, vorausgesetzt, daß Q ein Antikörper oder Antikörperfragment ist, wenn B ein Enzym ist, und daß Q ein Enzym ist, wenn B ein Antikörper oder Antikörperfragment ist; und
r gleich zwei bis fünfzig ist.

12. Das Konjugat nach Anspruch 11, worin B ein Enzym ist.

13. Ein Festphasenkonjugat umfassed: 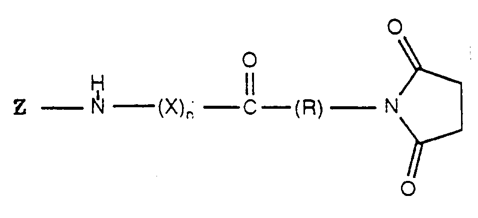 worin Z ein amintragendes Festphasenmaterial ist; X eine Aminosäure mit drei bis zehn Kohlenstoffatomen in einer geraden Kette ist; n gleich eins bis zehn ist und R entweder ein Alkyl mit eins bis sechs Kohlenstoffatomen, Cycloalkyl, Alkyl-cycloalkyl, worin das Alkyl eins bis sechs Kohlenstoffatome enthält, oder Phenyl ist.

14. Ein Festphasenkonjugat nach Anspruch 13, worin R Cyclohexylmethyl ist.

15. Ein Konjugat der Formel: 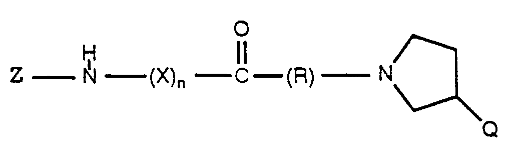 worin Q ein -SH oder thioltragendes Peptid, Polypeptid oder Protein ist, oder ein Peptid, Polypeptid oder Protein ist, das mit Sulfhydrylgruppen derivatisiert worden ist; Z ein amintragendes Festphasenmaterial ist; X eine Aminosäure mit drei bis zehn Kohlenstoffatomen in einer geraden Kette ist; n gleich eins bis zehn ist; und R entweder ein Alkyl mit eins bis sechs Kohlenstoffatomen, Cycloalkyl, Alkylcycloalkyl, worin das Alkyl eins bis sechs Kohlenstoffatome enthält, oder Phenyl ist.

16. Das Konjugat nach Anspruch 15, worin Z ein amintragendes Mikroteilchen ist.

## Patentansprüche (Patentansprüche für folgende(n) Vertragsstaat(en): ES)

1. Ein Verfahren Zur Darstellung eines heterofunktionellen Reagens, umfassed eine Verbindung mit der Formel: 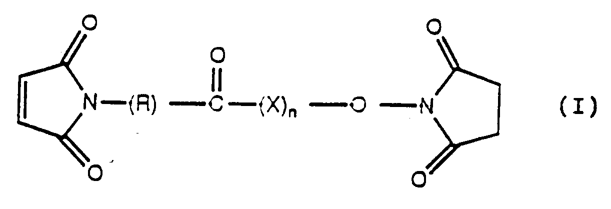 worin X eine Aminosäure mit drei bis zehn Kohlenstoffatomen in einer geraden Kette ist;
R entweder ein Alkyl mit einem bis sechs Kohlenstoffatomen, Cycloalkyl, Alkyl-cycloalkyl, worin das Alkyl aus eins bis sechs Kohlenstoffatomen besteht, oder ein Phenyl ist; und
n gleich eins bis zehn ist;
wobei das Verfahren die Umsetzung einer Verbindung mit der Formel: 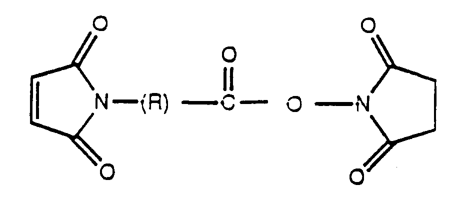 umfaßt, oder deren entsprechenden Säure mit der Aminosäure X und nachfolgende Aktivierung und Substitution der endständigen Säure der resultierenden Verbindung zur Bildung eines aktiven Esters nach Formel (I), worin n gleich eins ist, worin ausgehend von der letztgenannten Verbindung die Schritte zur Einführung einer Aminosäure X und die Synthese eines aktiven Esters ein bis neun Mal wiederholt werden können, zur Darstellung einer Verbindung (I), worin n gleich zwei bis zehn ist.

2. Das Verfahren nach Anspruch 1, worin R Cyclohexylmethyl ist.

3. Das Verfahren nach Anspruch 1, worin ausgehend von der Verbindung nach Formel (I), worin n gleich eins ist, die Schritte zur Einführung einer Aminosäure X und die Synthese eines aktiven Esters ein bis vier Mal wiederholt werden können zur Darstellung einer Verbindung (I), worin n gleich zwei bis fünf ist.

4. Das Verfahren nach Anspruch 1, worin ausgehend von der Verbindung nach Formel (I), worin n gleich eins ist, die Schritte zur Einführung einer Aminosäure X und die Synthese eines aktiven Esters zweimal wiederholt werden können zur Darstellung einer Verbindung (I), worin n gleich drei ist.

5. Das Verfahren nach den Ansprüchen 2 bis 4, worin X gleich 6-Aminocapronsäure ist.

6. Ein Verfahren für die Herstellug eines Konjugats für die Verwendung in einem Diagnoseassay, umfassed: die Kupplung eines Enzymmarkierers, Antikörpers oder Antikörperfragments an eine Verbindung entsprechend der folgenden Formel: 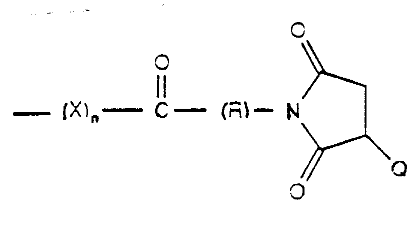 worin
Q ein -SH oder thioltragendes Peptid, Polypeptid oder Protein ist, oder ein Peptid, Polypeptid oder Protein ist, das mit Sulfhydrylgruppen derivatisiert worden ist, vorausgesetzt daß, wenn die Verbindung an einen Antikörper oder an ein Antikörperfragment gekuppelt ist, Q ein Enzym ist, und daß, wenn die Verbindung an einen Enzymmarkierer gekuppelt ist, Q ein Antikörper oder Antikörperfragment ist;
X eine Aminosäure mit drei bis zehn Kohlenstoffatomen in einer geraden Kette ist;
R ein Alkyl mit eins bis sechs Kohlenstoffatomen, Cycloalkyl, Alkyl-cycloalkyl, worin das Alkyl eins bis sechs Kohlenstoffatome enthält, oder ein Phenyl ist; und
n gleich eins bis zehn ist.

7. Ein Verfahren zur Darstellung eines Immunogens, umfassed die Kupplung einer Polyaminosäure an eine Verbindung entsprechend der folgenden Formel: 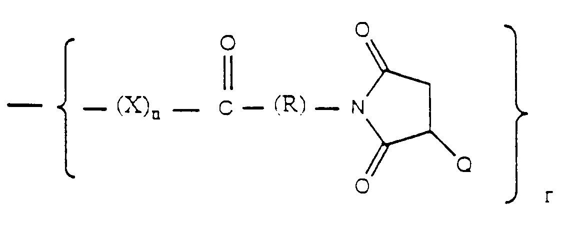 worin
X eine Aminosäure mit drei bis zehn Kohlenstoffatomen in einer geraden Kette ist;
R entweder ein Alkyl mit eins bis sechs Kohlenstoffatomen, Cycloalkyl, Alkyl-cycloalkyl, worin das Alkyl eins bis sechs Kohlenstoffatome enthält, oder Phenyl ist;
n gleich eins bis zehn ist;
r gleich 1 bis 20 ist; und
Q ein Hapten ist, gewählt aus einem -SH oder thioltragenden Peptid, Polypeptid oder Protein, oder einem Peptid, Polypeptid oder Protein, das mit Sulfhydrylgruppen derivatisiert worden ist.

8. Ein Verfahren zur Darstellung eines Konjugats für die Verwendung in einem Diagnoseassay, umfassed die Kupplung eines Enzyms, Antikörpers oder Antikörperfragments an eine Verbindung entsprechend der folgenden Formel: 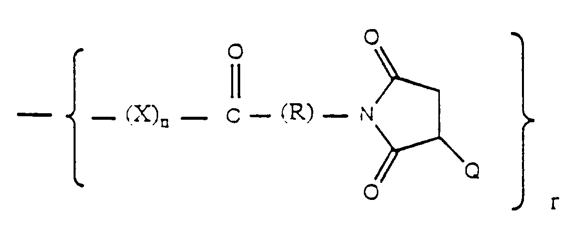 worin
X eine Aminosäure mit drei bis zehn Kohlenstoffatomen in einer geraden Kette ist;
R ein entweder Alkyl mit eins bis sechs Kohlenstoffatomen, Cycloalkyl, Alkyl-cycloalkyl, worin das Alkyl eins bis sechs Kohlenstoffatome enthält, oder ein Phenyl ist;
n gleich eins bis zehn ist
r gleich zwei bis fünfzig ist; und
Q ein -SH oder thioltragendes Peptid, Polypeptid oder Protein ist, oder ein Peptid, Polypeptid oder Protein ist, das mit Sulfhydrylgruppen derivatisiert worden ist, vorausgesetzt, daß Q ein Antikörper oder Antikörperfragment ist, wenn die Verbindung an ein Enzym gekuppelt ist, und daß Q ein Enzym ist, wenn die Verbindung an einen Antikörper oder an ein Antikörperfragment gekuppelt ist.

9. Ein Verfahren für die Darstellung eines Festphasenkonjugats, umfassed die Kupplung eines amintragenden Festphasenmaterials an eine Verbindung entsprechend der folgenden Formel: 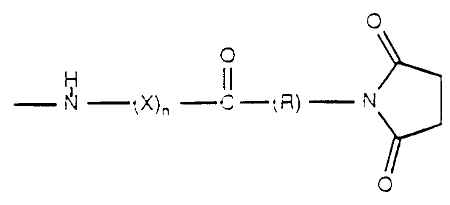 worin
X eine Aminosäure mit drei bis zehn Kohlenstoffatomen in einer geraden Kette ist;
n gleich eins bis zehn ist; und
R entweder ein Alkyl mit eins bis sechs Kohlenstoffatomen, Cycloalkyl, Alkyl-cycloalkyl, worin das Alkyl eins bis sechs Kohlenstoffatome enthält, oder ein Phenyl ist.

10. Ein Verfahren für die Darstellung eines Festphasenkonjugats, umfassend die der Kupplung eines Amintragenden Festphasenmaterials an eine Verbindung entsprechend der folgenden Formel: 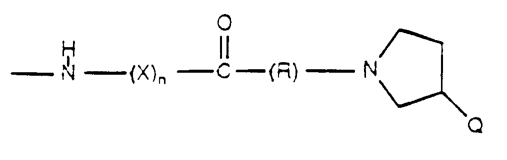 worin
Q ein -SH oder thioltragendes Peptid, Polypeptid oder Protein ist, oder ein Peptid, Polypeptid oder Protein ist, das mit Sulfhydrylgruppen derivatisiert worden ist;
X eine Aminosäure mit drei bis zehn Kohlenstoffatomen in einer geraden Kette ist;
n gleich eins bis zehn ist; und
R entweder ein Alkyl mit eins bis sechs Kohlenstoffatomen, Cycloalkyl, Alkyl-cycloalkyl, worin das Alkyl eins bis sechs Kohlenstoffatome enthält, oder ein Phenyl ist.

## Revendications (Revendications pour l'(les) Etat(s) contractant(s) suivant(s): AT, BE, CH, DE, FR, GB, GR, IT, LI, LU, NL, SE)

1. Réactif hétérobifonctionnel comprenant: 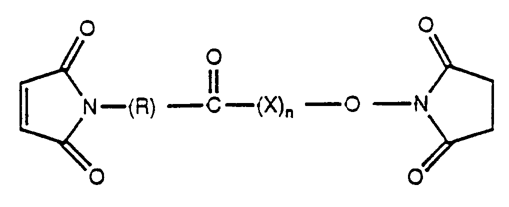 où X est un aminoacide ayant trois à dix atomes de carbone en chaîne linéaire;
R est un alkyle de un à six atomes de carbone, un cycloalkyle, un alkyle-cycloalkyle dans lequel ledit alkyle a un à six atomes de carbone, ou un phényle; et
n est compris entre un et dix.

2. Réactif selon la revendication 1, dans lequel R est un cyclohexylméthyle.

3. Réactif selon la revendication 1, dans lequel n est compris entre deux et cinq.

4. Réactif selon la revendication 3, dans lequel n est trois.

5. Réactif selon la revendication 1, dans lequel (X)ₙ est un polyamide formé d'unités répétées d'acide 6-aminoaproïque.

6. Conjugué à utiliser dans une analyse diagnostique, comprenant: 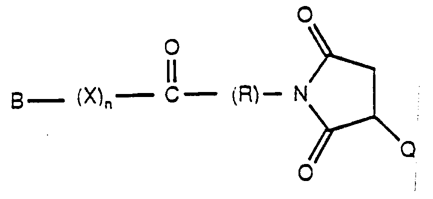 où B est un marqueur enzymatique, un anticorps, ou un fragment d'anticorps;
Q est un peptide, un polypeptide ou une protéine portant un groupe -SH ou thiol, ou un peptide, un polypeptide ou une protéine transformé(e) avec des groupes sulfhydryle, sous réserve que, lorsque B est un anticorps ou un fragment d'anticorps, Q soit un marqueur enzymatique, et que, lorsque B est un marqueur enzymatique, Q soit un anticorps ou un fragment d'anticorps;
X est un aminoacide ayant trois à dix atomes de carbone en chaîne linéaire;
R est un alkyle de un à six atomes de carbone, un cycloalkyle, un alkyle-cycloalkyle dans lequel ledit alkyle a un à six atomes de carbone, ou un phényle; et
n est compris entre un et dix.

7. Conjugué selon la revendication 6, dans lequel n est compris entre deux et cinq.

8. Conjugué selon la revendication 6, dans lequel (X)ₙ est un polyamide formé d'unités répétées d'acide 6-aminocaproïque.

9. Conjugué selon la revendication 6, dans lequel Q est une enzyme.

10. Conjugué à utiliser dans la préparation d'un immunogène, comprenant: 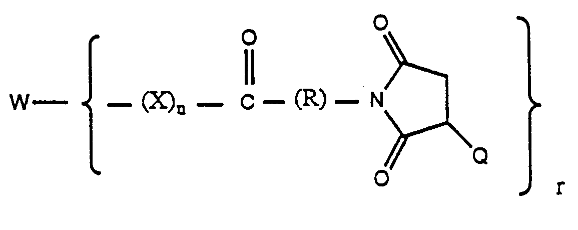 où X est un aminoacide ayant trois à dix atomes de carbone en chaîne linéaire;
R est un alkyle de un à six atomes de carbone, un cycloalkyle, un alkyle-cycloalkyle dans lequel ledit alkyle a un à six atomes de carbone, ou un phényle;
n est compris entre un et dix;
Q est un haptène choisi parmi un peptide, un polypeptide ou une protéine portant un groupe -SH ou thiol, ou un peptide, un polypeptide ou un protéine transformé(e) avec des groupes sulfhydryle;
W est un polyaminoacide; et
r est compris entre 1 et 20.

11. Conjugué à utiliser dans une analyse diagnostique, comprenant: 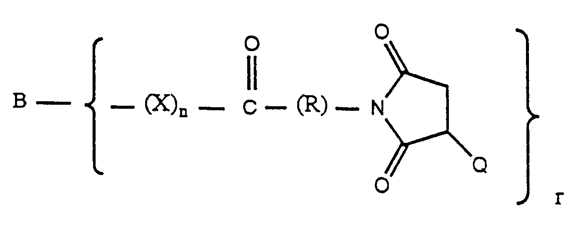 où X est un aminoacide ayant trois à dix atomes de carbone en chaîne linéaire;
R est un alkyle de un à six atomes de carbone, un cycloalkyle, un alkyle-cycloalkyle dans lequel ledit alkyle a un à six atomes de carbone, ou un phényle;
n est compris entre un et dix;
B est une enzyme, un anticorps ou un fragment d'anticorps;
Q est un peptide, un polypeptide ou une protéine portant un groupe -SH ou thiol, ou un peptide, un polypeptide ou une protéine transformé(e) avec des groupes sulfhydryle, sous réserve que Q soit un anticorps ou un fragment d'anticorps lorsque B est une enzyme, et que Q soit une enzyme lorsque B est un anticorps ou un fragment d'anticorps; et
r est compris entre deux et cinquante.

12. Conjugué selon la revendication 11, dans lequel B est une enzyme.

13. Conjugué en phase solide comprenant 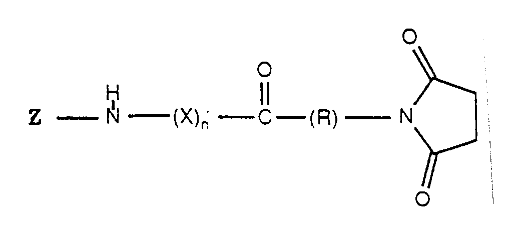 où Z est une matière en phase solide portant une amine;
X est un aminoacide ayant trois à dix atomes de carbone en chaîne linéaire;
n est compris entre un et dix; et
R est un alkyle de un à six atomes de carbone, un cycloalkyle, un alkyle-cycloalkyle dans lequel ledit alkyle a un à six atomes de carbone, ou un phényle.

14. Conjugué en phase solide selon la revendication 13, dans lequel R est un cyclohexylméthyle.

15. Conjugué de formule 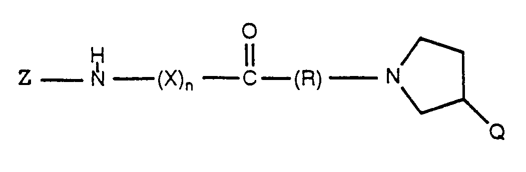 dans laquelle
Q est un peptide, un polypeptide ou une protéine portant un groupe -SH ou thiol, ou un peptide, un polypeptide ou une protéine transformé(e) avec des groupes sulfhydryle;
Z est une matière en phase solide portant une amine;
X est un aminoacide ayant trois à dix atomes de carbone en chaîne linéaire;
n est compris entre un et dix; et
R est un alkyle de un à six atomes de carbone, un cycloalkyle, un alkyle-cycloalkyle dans lequel ledit alkyle a un à six atomes de carbone, ou un phényle.

16. Conjugué selon la revendication 15, dans lequel Z est une microparticule portant une amine.

## Revendications (Revendications pour l'(les) Etat(s) contractant(s) suivant(s): ES)

1. Procédé de préparation d'un réactif hétérobifonctionnel comprenant un composé de formule: 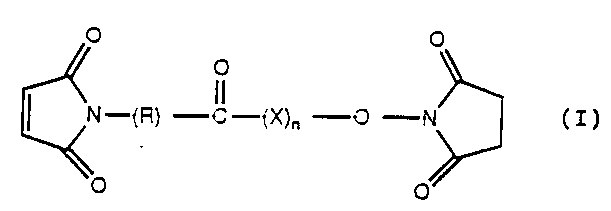 où X est un aminoacide ayant trois à dix atomes de carbone en chaîne linéaire;
R est un alkyle de un à six atomes de carbone, un cycloalkyle, un alkyle-cycloalkyle dans lequel ledit alkyle a un à six atomes de carbone, ou un phényle; et
n est compris entre un et dix;
ledit procédé comprenant les étapes selon lesquelles on fait réagir un composé de formule 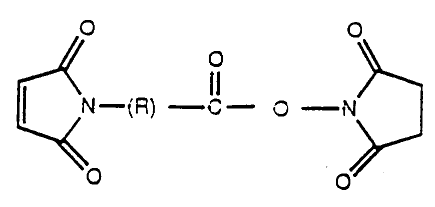 ou l'acide correspondant avec ledit aminoacide X, puis on active et on substitue l'acide terminal du composé obtenu pour former un ester actif de formule (I) dans laquelle n est un, et, à partir de ce dernier composé, on peut répéter de un à neuf fois les étapes d'insertion d'un aminoacide X et de synthèse d'un ester actif pour produire un composé (I) dans lequel n est de façon correspondante compris entre deux et dix.

2. Procédé selon la revendication 1, dans lequel R est un cyclohexylméthyle.

3. Procédé selon la revendication 1, dans lequel, en partant du composé de formule (I) dans laquelle n est un, on répète une à quatre fois les étapes d'insertion d'un aminoacide X et de synthèse d'un ester actif pour produire un composé (I) dans lequel n est de façon correspondante compris entre deux et cinq.

4. Procédé selon la revendication 1, dans lequel, en partant du composé de formule (I) dans laquelle n est un, on répète deux fois les étapes d'insertion d'un aminoacide X et de synthèse d'un ester actif pour produire un composé (I) dans lequel n est trois.

5. Procédé selon les revendications 2 à 4, dans lequel X est l'acide 6-aminocaproïque.

6. Procédé de préparation d'un conjugué à utiliser dans une analyse diagnostique, comprenant le couplage d'un marqueur enzymatique, d'un anticorps ou d'un fragment d'anticorps à un composé correspondant à la formule suivante: 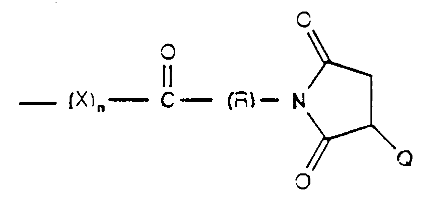 dans laquelle
Q est un peptide, un polypeptide ou une protéine portant un groupe -SH ou thiol, ou un peptide, un polypeptide ou une protéine transformé(e) avec des groupes sulfhydryle, sous réserve que, lorsque ledit composé est couplé à un anticorps ou un fragment d'anticorps, Q soit un marqueur enzymatique, et que, lorsque ledit composé est couplé à un marqueur enzymatique, Q soit un anticorps ou un fragment d'anticorps;
X est un aminoacide ayant trois à dix atomes de carbone en chaîne linéaire;
R est un alkyle de un à six atomes de carbone, un cycloalkyle, un alkyle-cycloalkyle dans lequel ledit alkyle a un à six atomes de carbone, ou un phényle; et
n est compris entre un et dix.

7. Procédé de préparation d'un immunogène, comprenant le couplage d'un polyaminoacide à un composé correspondant à la formule suivante: 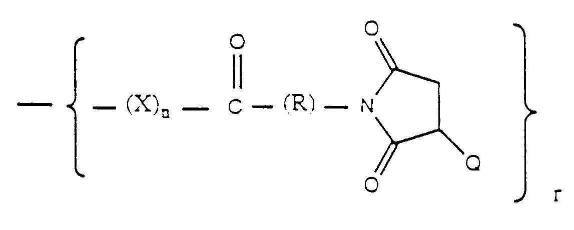 dans laquelle
X est un aminoacide ayant trois à dix atomes de carbone en chaîne linéaire;
R est un alkyle de un à six atomes de carbone, un cycloalkyle, un alkyle-cycloalkyle dans lequel ledit alkyle a un à six atomes de carbone, ou un phényle;
n est compris entre un et dix;
r est compris entre 1 et 20; et
Q est un haptène choisi parmi un peptide, un polypeptide ou une protéine portant un groupe -SH ou thiol, ou un peptide, un polypeptide ou une protéine transformé(e) avec des groupes sulfhydryle;

8. Procédé de préparation d'un conjugué à utiliser dans une analyse diagnostique, comprenant le couplage d'une enzyme, d'un anticorps ou d'un fragment d'anticorps à un composé correspondant à la formule suivante: 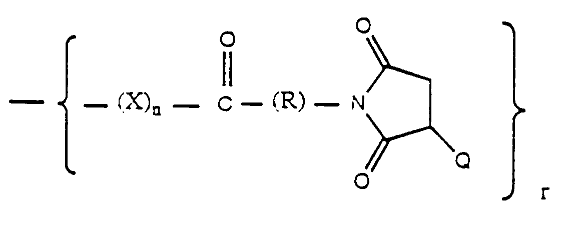 dans laquelle
X est un aminoacide ayant trois à dix atomes de carbone en chaîne linéaire;
R est un alkyle de un à six atomes de carbone, un cycloalkyle, un alkyle-cycloalkyle dans lequel ledit alkyle a un à six atomes de carbone, ou un phényle;
n est compris entre un et dix;
r est compris entre deux et cinquante; et
Q est un peptide, un polypeptide ou une protéine portant un groupe -SH ou thiol, ou un peptide, un polypeptide ou une protéine transformé(e) avec des groupes sulfhydryle, sous réserve que Q soit un anticorps ou un fragment d'anticorps lorsque ledit composé est couplé à une enzyme, et que Q soit une enzyme lorsque ledit composé est couplé à un anticorps ou un fragment d'anticorps.

9. Procédé de préparation d'un conjugué en phase solide, comprenant le couplage d'une matière en phase solide portant une amine à un composé correspondant à la formule suivante: 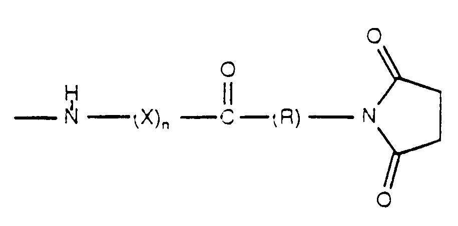 dans laquelle
X est un aminoacide ayant trois à dix atomes de carbone en chaîne linéaire;
n est compris entre un et dix; et
R est un alkyle de un à six atomes de carbone, un cycloalkyle, un alkyle-cycloalkyle dans lequel ledit alkyle a un à six atomes de carbone, ou un phényle.

10. Procédé de préparation d'un conjugué en phase solide comprenant le couplage d'une matière en phase solide portant une amine à un composé correspondant à la formule suivante: 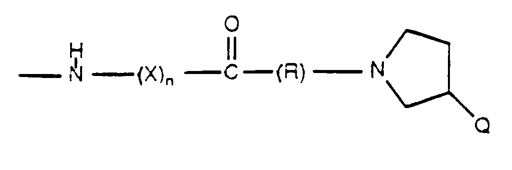 dans laquelle
Q est un peptide, un polypeptide ou une protéine portant un groupe -SH ou thiol, ou un peptide, un polypeptide ou une protéine transformé(e) avec des groupes sulfhydryle;
X est un aminoacide ayant trois à dix atomes de carbone en chaîne linéaire;
n est compris entre en et dix; et
R est un alkyle de un à six atomes de carbone, un cycloalkyle, un alkyle-cycloalkyle dans lequel ledit alkyle a un à six atomes de carbone, ou un phényle.
